# EUROPEAN PATENT SPECIFICATION

(11) **EP 1 228 095 B1**
(45) Date of publication and mention of the grant of the patent: **09.04.2008**
(21) Application number: 00971185.4
(22) Date of filing: 20.10.2000
(51) Int. Cl.: C07K 14/705, C12N 9/64, A61K 39/00, C12N 5/06, C12N 7/00, A61K 35/76, A61P 35/00, A61K 48/00

(54) **MODIFIED GP100 AND USES THEREOF**
MODIFIZIERTES GP100 UND DESSEN VERWENDUNG
MOLECULE GP100 MODIFIEE ET SES APPLICATIONS

(30) Priority: 22.10.1999 US 160879 P; 07.08.2000 US 223325 P
(43) Date of publication of application: 07.08.2002
(60) Divisional application: 07000096.3
(73) Proprietor: Sanofi Pasteur Limited, Toronto, Ontario M2R 3T4 (CA)
(72) Inventor: BERINSTEIN, Neil, Toronto, Ontario M5P 1V1 (CA); TARTAGLIA, James, Schenectady, NY 12303 (US); MOINGEON, Philippe, F-69480 Pommiers (FR); BARBER, Brian, Mississauga, Ontario L5G 3T5 (CA); TINE, John, A., Scotia, NY 12302 (US)
(74) Representative: Lawrence, Malcolm Graham
(86) International application number: PCT/CA2000/001254
(87) International publication number: WO 2001/030847

(56) References cited:
- WO-A-98/02538
- WO-A-98/04728
- WO-A-99/46988
- WO-A-99/46992
- IRVINE KARI R ET AL: "Recombinant virus vaccination against "self" antigens using anchor-fixed immunogens." CANCER RESEARCH, vol. 59, no. 11, 1 June 1999 (1999-06-01), pages 2536-2540, XP002161590 ISSN: 0008-5472
- PARKHURST M R ET AL: "Improved induction of melanoma-reactive CTL with peptides from the melanoma antigen gp100 modified at HLA-A*0201-binding residues." JOURNAL OF IMMUNOLOGY, (1996 SEP 15) 157 (6) 2539-48. , XP002096010
- KAMMULA U.S. ET AL: "Cancer immunotherapy: Is there real progress at last?." BIODRUGS, (1999) 11/4 (249-260). , XP000982586
- LILJEQVIST S.; STAHL S.: 'Production of recombinant subunit vaccines: protein immunogens, live delivery systems and nucleic acid vaccines' JOURNAL OF BIOTECHNOLOGY vol. 73, 30 July 1999, pages 1 - 33
- MARCHAND M. ET AL: 'Tumor regressions observed in patients with metastatic melanoma treated with an antigenic peptide encoded by gene MAGE-3 and presented by HLA-A1' INT. J. CANCER vol. 80, 18 January 1999, pages 219 - 230
- SPANER D. ET AL: 'Enhanced viral and tumor immunity with intranodal injection of canary pox viruses expressing the melanoma antigen, gp100' AMERICAN CANCER SOCIETY vol. 106, no. 4, 15 February 2006, pages 890 - 899

## Description

### FIELD OF THE INVENTION

The present invention is in the field of immunotherapy and relates to the construct of novel forms of gp100 suitable for the generation of immune responses *in vivo.*

### BACKGROUND OF THE INVENTION

The incidence and mortality of cutaneous malignant melanoma have risen dramatically over the past several decades (Liu, T. et al. (1996) Surg Clin North Am 76:1205; Gloster, H.M. et al. (1996) Dermatol Surg 22:217). At present, the estimated lifetime risk for an American of developing melanoma is approximately 1 in 90 (Rigel, D.S. et al. (1979) Mayo Clin Proc 72:367). While most early stage melanomas can be treated successfully by a simple surgical excision (Greenstein D.S. et al. (1995) Dermatol Surg 21:927; Whooley, B.P. et al. (1995) Dermatol Surg 4:187; Urist, M.M. et al. (1996) Ann Rev Med 47:211), patients with advanced disease are rarely cured even with aggressive chemotherapy and/or immunotherapy (Falkson, C.I. et al. (1995) Anticancer Drugs 6:709).

Although melanoma can present as an aggressive primary lesion that metastasizes within weeks, its development typically takes place over a period of several months to years and progresses through a series of distinct pathological stages. The earliest stage is the radial growth phase (RGP) melanoma that starts as an intradermal neoplasm (melanoma *in situ*). RGP melanomas may remain relatively quiescent for months or even years and are generally cured by simple excision. Eventually, most RGP melanomas develop a component of vertical growth (vertical growth phase melanoma, VGP), which denotes a more aggressive tumor less likely to be cured by simple excision. Metastatic spread is the final stage in tumor progression and is indicative of a very poor outcome (Reintgen, D. (1997) Ann Surg 225:1).

The most important prognostic factor in determining survival in a patient with primary melanoma is the depth of the lesion. While the five-year survival for patients with tumours of ≤0.75 mm in thickness is approximately 96%, individuals with tumours >4.5 mm in depth have a five year survival of only approximately 38% (Whooley, B.P. et al. (1995) Dermatol Surg 4:187; Urist, M.M. et al. (1996) Ann Rev Med 47:211). Since the development of a VGP melanoma from its RGP precursor generally takes at least several months, a "window of time" exists within which surgical excision can have a dramatic effect on patient outcome. By extension, it is reasonable to propose that novel attempts to deal with residual disease would be most successful at this time.

Several lines of evidence suggest that manipulation of immune responses against melanoma may be therapeutic: 1. Clinical observations of spontaneous regression of metastatic melanoma may be caused by an anti-melanoma immune response (Nelson, C.A. et al. (1976) Natl Cancer Inst Monogr 44:145). 2. Regression of metastatic melanoma has also been observed in some patients given high doses of IL-2 with or without lymphokine activated killer (LAK) cells or tumor infiltrating lymphocytes (TIL's) (Rosenberg, S.A. et al. (1998) J Natl Cancer Inst 90:1894) 3. More recently, a number of melanoma specific and associated tumor antigens have been cloned (Van den Eynde, B. et al. (1997) Curr Opin Immunol 9:684). The availability of these reagents has given hope that specific vaccines may be developed to enhance the ability of tumor specific T cells to eliminate melanoma cells. These antigens can be administered in a variety of delivery vehicles, and the most effective dosing regimen for optimization of an anti-tumor response is not presently clear.

Conventional vaccines for many infectious diseases have shown that a primary infectious challenge can induce an effective protective memory immune response. A critical event required to initiate an immune response, even in the presence of circulating tumor reactive T cells, is that tumor antigens gain entry into the secondary lymphoid structures of the spleen and lymph nodes (Zinkernagel, R.M.et al. (1997) Immunol Rev 156:199). Melanoma that develops locally in the skin is hidden from the immune system as long as the tumor antigens do not reach the secondary lymphoid organs. The tumor may then grow to such a large mass that, by the time an immune response is triggered, it is rapidly overwhelmed by the tumor (Moskophiis, D. et al. (1993) Nature 362:758). Alternatively, melanoma cells that break off and traffic to the local lymph node may not be able to trigger an immune response because they are poor antigen presenting cells, i.e., they do not express high levels of HLA-class I molecules (Ferrone, S. (1995) Immunology Today 61:487) or co-stimulatory molecules, such as members of the B7 family (Bluestone, J.A. et al. (1995) Immunity 2:555). Again, the tumor may reach a tolerizing size before an immune response is initiated. A successful vaccination strategy in patients with deep, but not metastatic, melanoma should both increase the number of tumor reactive T cells and activate them so that they can traffic to the periphery and exert their cytotoxic effector function.

gp100 is normally found in melanosomes and expressed in melanocytes, retinal cells, and other neural crest derivatives (Kawakami, Y. et al. (1997) Int Rev Immunol 14:173*).* The function of gp100 is currently unknown (Rosenberg, S.A. et al. (1998) Nature Med 4:321). By mass spectrometry, three immunodominant HLA-A2 binding gp100 peptides have been identified: g9-154 (amino acids 154-162), g9-209 (amino acids 209-217); and g9-280 (amino acids 280-288) (Kawakami, Y. et al. (1995) J Immunol 154:3961). Notably, two of these peptides have been synthetically altered so as to induce a more vigorous immune response in the original T cell clone: the threonine at position 2 in g9-209 was changed to a methionine, and the alanine residue at position 9 in g9-280 was changed to a valine (Parkhurst, M.R. et al. (1996) J Immunol 157:2539). These changes increase the binding affinity of the peptides to the HLA-A2 molecule without changing the epitopes recognized by the T cell receptor (TCR). Rosenberg et al have already successfully immunized melanoma patients with one of these modified peptides and achieved objective clinical responses in some patients (Rosenberg, S.A. et al. (1998) Nature Med 4:321).

A poxviral vector comprising a nucleic acid encoding gp100 modified at codons 210 (T21OM) and 288 (A288V) has been shown to induce a gp100 specific immune response in mice (Irvine, K et al., (1999) Cancer Research 59:2536).

### SUMMARY OF THE INVENTION

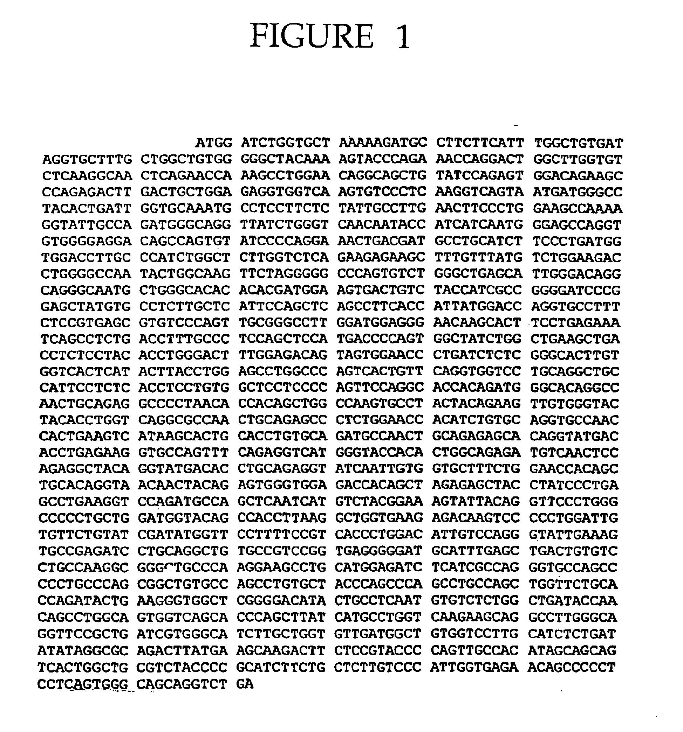

The present inventors have developed a pharmaceutical composition comprising a poxvirus which has inserted therein a nucleic acid sequence encoding *gp* 100M as shown in figure 1, defined by SEQ ID NO:1, for priming, and the peptides consisting of amino acids sequences according to SEQ ID NO:124 and SEQ ID NO:125 for boosting, wherein the composition is adapted for separate administration of the vector and the peptides. This composition may be used for producing an immune response to human melanoma by sequential administration.

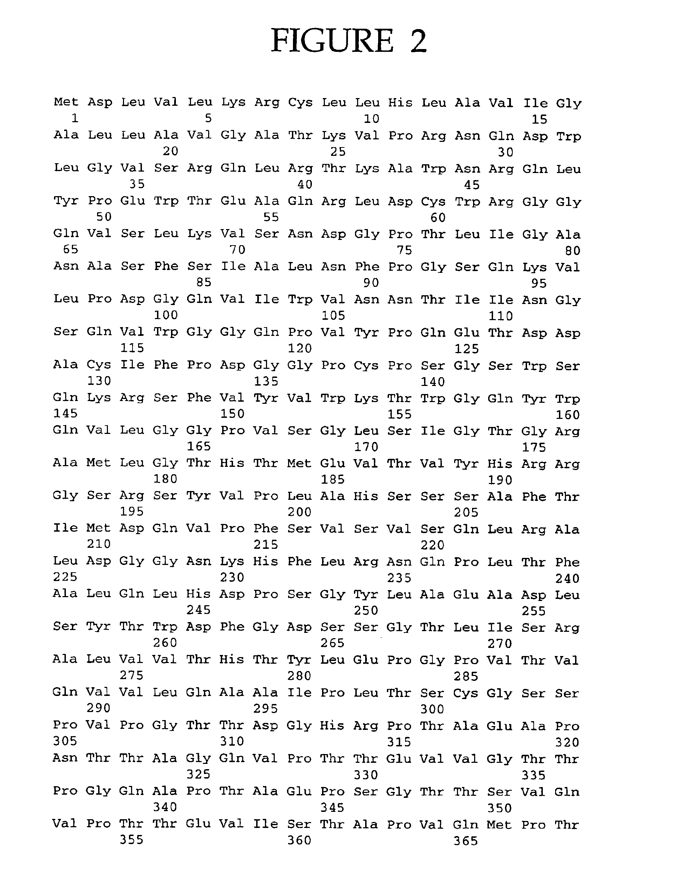

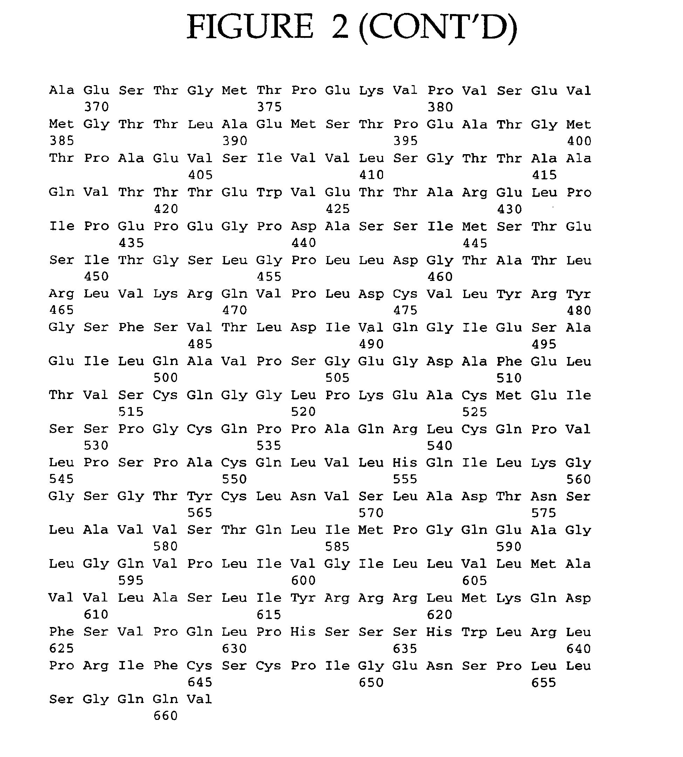

The nucleic acid sequence of *gp100M* is shown in Figure 1 and is also known herein as SEQ.ID.NO.1. The corresponding amino acid sequence encoded by the nucleic acid sequence of *gp100M* is shown in Figure 2 which is referred to herein as SEQ.ID.NO.2.

There is described an isolated nucleic acid molecule is provided having a sequence as shown in Figure 1 (SEQ.ID.NO.1).

Preferably, the purified and isolated nucleic acid molecule comprises:
(a) a nucleic acid sequence as shown in SEQ.ID.NO.1 wherein T can also be U;
(b) nucleic acid sequences complementary to (a);
(c) nucleic acid sequences which are homologous to (a) or (b);
(d) a fragment of (a) to (c) that is at least 15 bases, preferably 20 to 30 bases, and which will hybridize to (a) to (d) under stringent hybridization conditions; or
(e) a nucleic acid molecule differing from any of the nucleic acids of (a) to (c) in codon sequences due to the degeneracy of the genetic code.

There is described an isolated gp100M protein and/or immunogenic fragments thereof encoded by a nucleic acid molecule of the invention. The gp100M has the amino acid as shown in Figure 2 (SEQ.ID.NO.2).

Preferred fragments include a fragment having an amino acid sequence according to SEQ.ID.NO.124 or according to SEQ.ID.NO.125.

There is also described method of modulating an animal's immune system comprising administering, to an animal in need thereof, an effective amount of a gp100 or *gp100* which has been modified to provide a molecule which modulates the immune system. Preferably the modified gp100 or *gp100* is gp100M or *gp100M,* respectively, most preferably the modified gp100 or *gp100* have the sequences of SEQ. ID. NO 1 and 2 respectively.

There is also described a method of modulating an animal's immune system comprising administering to an animal in need thereof, an effective amount of a vector into which has been inserted a *gp100* which has been modified to provide a molecule which modulates the immune system, preferably the vector is viral, preferably the virus is an adenovirus, alphavirus or poxvirus. More preferably where the virus is poxvirus it is vaccinia, fowlpox, avipox, TROVAC, ALVAC, NYVAC or MVA, preferably ALVAC..

The modified gp100 or *gp100* may be administered with a second agent, preferably a lymphokine, cytokine, or co-stimulatory molecule such as a member of the B7 family of molecules, preferably the cytokine is GM-CSF, IL-2, IL-12, TNF or IFNγ1.

There is described a method of stimulating an animal's immune system comprising administering to an animal in need thereof, an effective amount of a gp100 or *gp100* which has been modified to provide a molecule which stimulates the immune system, preferably gp100M or *gp100M,* respectively, most preferably the modified gp100 or *gp100* have the sequences of SEQ. ID. NO 1 and 2 respectively

There is described a method of enhancing the efficacy of a gene vaccine in the treatment of an animal comprising administering an effective amount of a *gp100* which has been modified to provide a molecule which stimulates the immune system, preferably *gp100M* ; For example, preferably when the animal has cancer.

There is described a composition for modulating an animal's immune system comprising an effective amount of a gp100 which has been modified to provide a molecule which stimulates the immune system, preferably gp100M, in a pharmaceutically acceptable diluent or carrier, most preferably the modified gp100 has the sequence of SEQ. ID. NO 1

There is described methods for prophylactic or therapeutic uses involving a nucleic acid sequence encoding a modified gp100, preferably gp100M, more preferably a gp100M having and amino acid sequence according to SEQ ID NO. 2.

There is also described a melanoma vaccine comprising a nucleic acid sequence encoding a modified gp100 for preventing or treating cancer preferably gp100M, more preferably a gp100M having and amino acid sequence according to SEQ ID NO. 2

There is also described a melanoma vaccine comprising a nucleic acid sequence encoding a modified gp100 for preventing or treating melanoma preferably gp100M, more preferably a gp100M having and amino acid sequence according to SEQ ID NO. 2.

There is described a modified gp100 protein sequence which is modified to increase its immunogenicity or to enhance its induction of an anti-melanoma immune response by enhancing the binding to MHC molecules, for use in a prophylactic or therapeutic methods described herein.

There is described a vaccine comprising a modified gp100 nucleic acid sequence or its corresponding protein capable of eliciting the production of antibodies in a mammal to corresponding antigens preferably *gp100M* or gp100M, respectively, more preferably a *gp100M* having and nucleic acid sequence according to SEQ ID NO. 1 and a gp100M having and amino acid sequence according to SEQ ID NO. 2..

There is described a antigenic, immunological or vaccine composition or a therapeutic composition for inducing an antigenic or immunological response in a host animal inoculated with the composition, said vaccine including a modified recombinant virus having inactivated nonessential virus-encoded genetic functions so that the recombinant virus has attenuated virulence and enhanced safety.

There is described an immunogenic composition containing a modified recombinant virus having inactivated nonessential virus-encoded genetic functions so that the recombinant virus has attenuated virulence and enhanced safety.

There is described a modified recombinant virus having nonessential virus-encoded genetic functions inactivated therein so that the virus has attenuated virulence, and wherein the modified recombinant virus further contains DNA from a heterologous source in a nonessential region of the virus genome.

There is described a recombinant virus comprising a virus into which is inserted a nucleic acid according to to the invention wherein the nucleic acid encodes for a polypeptide, the recombinant virus causing the expression of the polypeptide in an infected cell.

There is described a recombinant virus into which is inserted a nucleic acid according to the present invention wherein the nucleic acid encodes for a modified gp100 polypeptide, wherein cells infected with the said recombinant virus are capable of eliciting an immune response directly against a member selected from the group consisting of:
(1) the polypeptide;
(2) a fragment of the polypeptide;
(3) a cell expressing the polypeptide or a fragment thereof; or
(4) cells binding the protein or fragment thereof, preferably the virus is adenovirus, alphavirus, or poxvirus, preferably where the virus is poxvirus it is vaccinia, fowlpox, avipox, TROVAC, ALVAC, NYVAC or MVA, preferably the virus is ALVAC.

Such recombinant virus' may be part of a composition comprising the particular recombinant virus and a pharmaceutically acceptable diluent or carrier.

There is also described the product of expression of a recombinant virus including a nucleic acid encoding a modified gp100, preferably the virus is an adenovirus, alphavirus or poxvirus, more preferably where the virus is poxvirus it is vaccinia, fowlpox, avipox, TROVAC, ALVAC, NYVAC or MVA, preferably ALVAC, and uses therefor, such as to form antigenic, immunological or vaccine compositions for treatment, prevention, diagnosis or testing; and, to DNA from the recombinant poxvirus which is useful in constructing DNA probes and PCR primers.

Other features and advantages of the present invention will become apparent from the following detailed description. It should be understood, however, that the detailed description and the specific examples while indicating preferred embodiments of the invention are given by way of illustration only.

### DESCRIPTION OF THE DRAWINGS

The invention will be better understood with reference to the drawing in which:
Figure 1 shows the nucleic acid sequence of *gp100M* cDNA.
Figure 2 shows the deduced amino acid sequence for the gp100M protein.
Figure 3 shows the nucleic acid sequence of C5H6gp100M, the H6 promoted human *gp100M* insertion cassette.
Figure 4 shows a schematic representation of the ALVAC(2)-gp100M(vCP1584) genome.
Figure 5 shows the nucleotide sequence of the oligonucleotide primers used to sequence pBS/1584.
Figure 6 shows immunoprecipitate results from uninfected HeLa cells or cells infected with either ALVAC parental virus, ALVAC-gp100, or ALVAC (2)-gp100M.
Figure 7 shows a Western blot of HeLa cells infected with one of ALVAC parental virus, ALVAC-gp100, or ALVAC (2)-gp100M, illustrating expression of full length gp100 in ALVAC-gp100, or ALVAC (2)-gp100M infected cells.
Figure 8 is a bar graph showing the results of an IFN-γ-ELISPOT analysis of animal receiving intranodal injection of the tumor antigen.
Figure 9 is a bar graph showing the results of an IFN-γ-ELISPOT analysis of animal receiving intranodal injection of the tumor antigen.
Figure 10 is a bar graph showing the results of an IFN-γ-ELISPOT analysis of animal receiving subcutaneous injection of the tumor antigen.
Figure 11 is a bar graph showing the results of an IFN-γ-ELISPOT analysis of animal receiving subcutaneous injection of the tumor antigen.
Figure 12 is a graph showing the antibody response after a regiment of intranodal (group 2) and subcutaneous (group 3) administration of ALVAC-modified gp100/modified gp100 peptide immunogens.

### DETAILED DESCRIPTION OF THE INVENTION

As already mentioned, the present inventors have developed a pharmaceutical composition comprising a poxvirus which has inserted therein a nucleic acid sequence encoding *gp*100M as shown in figure 1, defined by SEQ ID NO:1, for priming, and the peptides consisting of amino acids sequences according to SEQ ID NO:124 and SEQ ID NO:125 for boosting, wherein the composition is adapted for separate administration of the vector and the peptides. This composition is useful for producing an immune response to human melanoma by sequential administration.

### I. NUCLEIC ACID MOLECULES EMPLOYED IN THE INVENTION

As mentioned above, the inventors have described and characterized the gene (*gp100M*) and its gene product (gp100M).

Broadly stated, there is described an isolated nucleic acid molecule comprising a sequence encoding a protein with the activity of a gp100 which has been modified to provide a molecule which stimulates the immune system. As used herein a gp100 which has been modified to provide a molecule which stimulates the immune system includes those gp100 sequences with modified sequences at about amino acids 209 and/or at about 280 (as set out in Parkhurst, M.R., et al. J. Immunol. 157:2539-2548 (1996)) and/or immunogenic fragments thereof.

There is described an isolated nucleic acid molecule encoding a modified gp100 capable of modulating the immune system. The term "isolated" refers to a nucleic acid substantially free of cellular material or culture medium when produced by recombinant DNA techniques, or chemical precursors, or other chemicals when chemically synthesized. The term "nucleic acid" is intended to include DNA and RNA and can be either double stranded or single stranded. An isolated nucleic acid molecule is described having a sequence as shown in Figure 1.

Preferably, the purified and isolated nucleic acid molecule comprises:
(a) a nucleic acid sequence as shown in Fig. 1, wherein T can also be U;
(b) nucleic acid sequences complementary to (a);
(c) nucleic acid sequences which are homologous to (a) or (b);
(d) a fragment of (a) to (c) that is at least 15 bases, preferably 20 to 30 bases, and which will hybridize to (a) to (c) under stringent hybridization conditions; or
(e) a nucleic acid molecule differing from any of the nucleic acids of (a) to (c) in codon sequences due to the degeneracy of the genetic code.

There is described nucleic acid molecules encoding truncations of the proteins of the invention, and analogs and homologs of the proteins of the invention and truncations thereof, as described below.

There is also described nucleic acid molecules comprising nucleic acid sequences having substantial sequence homology with the nucleic acid sequence as shown in Figure 1 and fragments thereof. The term "sequences having substantial sequence homology" means those nucleic acid sequences which have slight or inconsequential sequence variations from these sequences, i.e., the sequences function in substantially the same manner to produce functionally equivalent proteins. The variations may be attributable to local mutations or structural modifications.

Generally, nucleic acid sequences having substantial homology include nucleic acid sequences having at least 70%, preferably 80-90% identity with the nucleic acid sequence as shown in Figure 1.

There is also described a nucleic acid molecule, and fragments thereof having at least 15 bases, which hybridize to the nucleic acid molecules of the invention under hybridization conditions, preferably stringent hybridization conditions. Appropriate stringency conditions which promote DNA hybridization are known to those skilled in the art (for example, Current Protocols in Molecular Biology, John Wiley & Sons, N.Y. (1989), 6.3.1-6.3.6). Hybridization procedures are also well known to those skilled in the art (for example, as described in Ausubel et al., Current Protocols in Molecular Biology, John Wiley & Sons Inc. (1994), Silhavy et al., Experiments with Gene Fusion, Cold Spring Harbor Laboratory Press (1984); Davis et al. Methods in Enzymol. 65:404 (1980)). Important parameters that can be considered for optimizing hybridization conditions are reflected in a formula that allows calculation of a critical value, the melting temperature above which two complementary DNA strands separate from each other (Davis et al. Methods in Enzymol. 65:404(1980)). This formula is as follows: Tm = 81.5 + 0.41 x (% G+C) + 16.6 log (actual ion concentration) - 0.63 x (% formamide) - 600/base number. Under appropriate stringency conditions, hybridization temperature (Th) is approximately 20 to 40°C, 20 to 25°C, or, preferably 30 to 40°C below the calculated Tm. Those skilled in the art will understand that optimal temperature and salt conditions can be readily determined empirically in preliminary experiments using conventional procedures.

For example, stringent conditions can be achieved, both for pre-hybridizing and hybridizing incubations, (i) within 4 to 16 hours at 42°C, in 6 x SSC containing 50% formamide or (ii) within 4-16 hours at 65°C in an aqueous 6 x SSC solution (1 M NaCl, 0.1 M sodium citrate (pH 7.0)). Typically, hybridization experiments are performed at a temperature from 60 to 68°C, e.g. 65°C. At such a temperature, stringent hybridization conditions can be achieved in 6xSSC, preferably in 2xSSC or 1xSSC, more preferably in 0.5xSSc, 0.3xSSC or 0.1xSSC (in the absence of formamide). 1xSSC contains 0.15 M NaCl and 0.015 M sodium citrate.

For polynucleotides containing 30 to 600 nucleotides, the above formula is used and then is corrected by subtracting (600/polynucleotide size in base pairs). Stringency conditions are defined by a Th that is 5 to 10°C below Tm.

Hybridization conditions with oligonucleotides shorter than 20 to 30 bases do not exactly follow the rules set forth above. In such cases, the formula for calculating the Tm is as follows: Tm = 4 x (G+C) + 2 (A+T). For example, an 18 nucleotide fragment of 50% G+C would have an approximate Tm of 54°C.

Nucleic acid molecules from a modified *gp100* gene such as the *gp100M* gene can be isolated by preparing a labeled nucleic acid probe based on all or part of the nucleic acid sequence as shown in Figure 1, and using this labeled nucleic acid probe to screen an appropriate DNA library (e.g. a cDNA or genomic DNA library). Nucleic acids isolated by screening of a cDNA or genomic DNA library can be sequenced by standard techniques.

Nucleic acid molecules as described can also be isolated by selectively amplifying a nucleic acid using the polymerase chain reaction (PCR) method and cDNA, genomic DNA or other source of DNA. It is possible to design synthetic oligonucleotide primers from the nucleic acid molecule as shown in Figure 1 for use in PCR. These synthetic oligonucleotide primers may also be further modified to incorporate specific changes from the normal nucleic acid sequence so as to be utilized for site directed mutagenesis. A nucleic acid can be amplified from cDNA or genomic DNA using these oligonucleotide primers and standard PCR amplification techniques. The nucleic acid so amplified can be cloned into an appropriate vector and characterized by DNA sequence analysis. It will be appreciated that cDNA may be prepared from mRNA, by isolating total cellular mRNA by a variety of techniques, for example, by using the guanidinium-thiocyanate extraction procedure of Chirgwin et al., (Biochemistry, 18, 5294-5299 (1979)). cDNA is then synthesized from the mRNA using reverse transcriptase (for example, Moloney MLV reverse transcriptase available from Gibco/BRL, Bethesda, MD, or AMV reverse transcriptase available from Seikagaku America, Inc., St. Petersburg, FL).

An isolated nucleic acid molecule as described which is RNA can be isolated by cloning a cDNA encoding a novel protein of the invention into an appropriate vector which allows for transcription of the cDNA to produce an RNA molecule which encodes the gp100M protein. For example, a cDNA can be cloned downstream of a bacteriophage promoter, (e.g., a T7 promoter) in a vector, cDNA can be transcribed *in vitro* with T7 polymerase, and the resultant RNA can be isolated by standard techniques.

A nucleic acid molecule as described may also be chemically synthesized using standard techniques. Various methods of chemically synthesizing polydeoxynucleotides are known, including solid-phase synthesis which, like peptide synthesis, has been fully automated in commercially available DNA synthesizers (See e.g., U.S. Patent No. 4,598,049; U.S. Patent No. 4,458,066; and U.S. Patent Nos. 4,401,796 and 4,373,071).

The initiation codon and untranslated sequences of the nucleic acid molecules as described may be determined using currently available computer software designed for the purpose, such as PC/Gene (IntelliGenetics Inc., Calif.). Regulatory elements can be identified using conventional techniques. The function of the elements can be confirmed by using these elements to express a reporter gene which is operatively linked to the elements. These constructs may be introduced into cultured cells using standard procedures. In addition to identifying regulatory elements in DNA, such constructs may also be used to identify proteins interacting with the elements, using techniques known in the art.

There is also described nucleic acids encoding fusion proteins comprising a protein as described and a selected protein, or a selectable marker protein (see below).

### II. METHODS OF EXPRESSING NUCLEIC ACID SEQUENCES FOR USE IN THE INVENTION

A polynucleotide molecule as described containing RNA, DNA, or modifications or combinations thereof, can have various applications. For example, a polynucleide molecule can be used (i) in a process for producing the encoded polypeptide in a recombinant host system, (ii) in the construction of vaccine vectors (such as poxviruses) used for modulating immune systems; (iii) as vaccine vectors which are further used in methods and compositions for preventing and/or treating melanoma, (iv) as a vaccine agent (as well as an RNA molecule), in a naked form or formulated with a delivery vehicle and, (v) as detection reagents/hybridization probes in molecular and/or therapeutic assays.

There are also described (i) an expression cassette containing a DNA molecule as described placed under the control of the elements required for expression, in particular under the control of an appropriate promoter; (ii) an expression vector containing an expression cassette as described; (iii) a procaryotic or eucaryotic cell transformed or transfected with an expression cassette and/or vector as described, as well as (iv) a process for producing a polypeptide or polypeptide derivative encoded by a polynucleotide as described, which involves culturing a procaryotic or eucaryotic cell transformed or transfected with an expression cassette and/or vector as described, under conditions that allow expression of the DNA molecule as described and, recovering the encoded polypeptide or polypeptide derivative from the cell culture.

A recombinant expression system can be selected from procaryotic and eucaryotic hosts. Eucaryotic hosts include yeast cells (e.g., *Saccharomyces cerevisiae* or *Pichia pastoris*), mammalian cells (*e.g.,* COS1, NIH3T3, or JEG3 cells), arthropods cells (e.g., *Spodoptera frugiperda* (SF9) cells), and plant cells. Preferably, a procaryotic host such as E. *coli* is used. Bacterial and eucaryotic cells are available from a number of different sources to those skilled in the art, e.g., the American Type Culture Collection (ATCC; 10801 University Blvd., Manassas, VA 20110-2209, USA).

The choice of the expression system depends on the features desired for the expressed polypeptide. For example, it may be useful to produce a polypeptide as described in a particular lipidated form or any other form.

The choice of the expression cassette will depend on the host system selected as well as the features desired for the expressed polypeptide. Typically, an expression cassette includes a promoter that is functional in the selected host system and can be constitutive or inducible; a ribosome binding site; a start codon (ATG) if necessary; a region encoding a signal peptide (*e.g.,* a lipidation signal peptide); a DNA molecule as described a stop codon; and optionally a 3' terminal region (translation and/or transcription terminator). The signal peptide-encoding region is adjacent to the polynucleotide as described and placed in proper reading frame. The signal peptide-encoding region can be homologous or heterologous to the DNA molecule encoding the mature polypeptide and can be specific to the secretion apparatus of the host used for expression. The open reading frame constituted by the DNA molecule as described solely or together with the signal peptide, is placed under the control of the promoter so that transcription and translation occur in the host system. Promoters, (and signal peptide encoding regions) are widely known and available to those skilled in the art and include, for example, the promoter of *Salmonella typhimurium* (and derivatives) that is inducible by arabinose (promoter araB) and is functional in Gram-negative bacteria such as *E. coli* (as described in U.S. Patent No. 5,028,530 and in Cagnon et al., Protein Eng. 4:843 (1991)), the promoter of the gene of bacteriophage T7 encoding RNA polymerase that is functional in a number of E. *coli* strains expressing T7 polymerase (described in U.S. Patent No. 4,952,496), OspA lipidation signal peptide, and the RIpB lipidation signal peptide (Cagnon et al., Protein Eng. 4:843 (1991)).

The expression cassette is typically part of an expression vector, which is selected for its ability to replicate in the chosen expression system. Expression vectors (*e.g.*, plasmids or viral vectors) can be chosen from those described in Pouwels et al. (Cloning Vectors: A Laboratory Manual 1985, Supp. 1987). They can be purchased from various commercial sources.

Methods for transforming/transfecting host cells with expression vectors will depend on the host system selected as described in Ausubel et al., Current Protocols in Molecular Biology, John Wiley & Sons Inc. (1994).

Upon expression, a recombinant polypeptide as described (or a polypeptide derivative) is produced and remains in the intracellular compartment, is secreted/excreted in the extracellular medium or in the periplasmic space, or is embedded in the cellular membrane. The polypeptide can then be recovered in a substantially purified form from the cell extract or from the supernatant after centrifugation of the recombinant cell culture. Typically, the recombinant polypeptide can be purified by antibody-based affinity purification or by any other method that can be readily adapted by a person skilled in the art, such as by genetic fusion to a small affinity binding domain. Antibody-based affinity purification methods are also available for purifying a polypeptide as described. Antibodies useful for purifying by immunoaffinity the polypeptides as described can be obtained as described below.

There is described (i) an expression cassette containing a DNA molecule as described under the control of the elements required for expression, in particular under the control of an appropriate promoter; (ii) an expression vector containing an expression cassette as described; (iii) a procaryotic or eucaryotic cell transformed or transfected with an expression cassette and/or vector as described, as well as (iv) a process for producing a polypeptide or polypeptide derivative encoded by a polynucleotide as described which involves culturing a procaryotic or eucaryotic cell transformed or transfected with an expression cassette and/or vector as described under conditions that allow expression of the DNA molecule as described, and recovering the encoded polypeptide or polypeptide derivative from the cell culture.

### III. PROTEINS FOR USE IN THE INVENTION

There is described an isolated protein encoded by nucleic acid molecules as described. Within this context, a protein as described may include various structural forms of the primary protein which retain biological activity. As used herein "modified gp100" or "modified gp100 protein", "gp100M" or "gp100M protein", means a gp100 which has been modified to provide a molecule which modulates the immune system. By "polypeptide" or "protein" is meant any chain of amino acids, regardless of length or post-translational modification (e.g., glycosylation or phosphorylation). Both terms are used interchangeably in the present application.

The terms "modified gp100", "modified gp100 protein", "gp100M" or "gp100M protein" as used herein is intended to include analogs of a modified gp100 or gp100M, containing one or more amino acid substitutions, insertions, and/or deletions. Amino acid substitutions may be of a conserved or non-conserved nature. Conserved amino acid substitutions involve replacing one or more amino acids with amino acids of similar charge, size, and/or hydrophobicity characteristics. When only conserved substitutions are made the resulting analog should be functionally equivalent to a modified gp100M. Non-conserved substitutions involve replacing one or more amino acids with one or more amino acids which possess dissimilar charge, size, and/or hydrophobicity characteristics.

One or more amino acid insertions may be introduced into the amino acid sequence of modified gp100, preferably gp100M. Amino acid insertions may consist of single amino acid residues or sequential amino acids.

Deletions may consist of the removal of one or more amino acids, or discrete portions (i.e. amino acids) from the gp100M amino acid sequence. The deleted amino acids may or may not be contiguous.

Also included in the expression "gp100M", "modified gp100", "modified gp100 protein" or "gp100M protein" as used herein are homologs of gp100M. Such homologs are proteins whose amino acid sequences are comprised of the amino acid sequences of gp100M regions from other sources whose coding nucleic acid sequences hybridize under stringent hybridization conditions (which conditions are known to those skilled in the art) with a nucleic acid probe used to obtain gp100M. It is anticipated that a protein comprising an amino acid sequence which is at least 72%, preferably 75 to 90% similar, with the amino acid sequence of gp100M will exhibit gp100M activity.

As used herein the expressions "modified gp100", "modified gp100 protein", "gp100M", or "gp100M protein" also contemplate isoforms of the modified gp100, or gp100M protein. An isoform contains the same number and kinds of amino adds as the modified gp100 or gp100M, but the isoform has a different molecular structure. The isoforms contemplated are those having the same properties as the protein described herein. Also included in the expression are other proteins which share similarities to a modified gp100 or gp100M.

Broadly stated, there is described an isolated protein with activity equivalent to that of a modified gp100, preferably a gp100M protein.

Preferably the protein has the amino acid sequence as shown in Figure 2.

In addition to full length amino acid sequences, the proteins as described also include truncations of the protein and analogs and homologs of the protein and truncations thereof as described herein. Truncated proteins may comprise peptides of at least fifteen amino acid residues. Analogs of the protein having the amino acid sequence shown in Figure 2 and/or truncations thereof as described herein, may include, but are not limited to, an amino acid sequence containing one or more amino acid substitutions, insertions, and/or deletions. Amino acid substitutions may be of a conserved or non-conserved nature. Conserved amino acid substitutions involve replacing one or more amino acids of the proteins of the invention with amino acids of similar charge, size, and/or hydrophobicity characteristics. When only conserved substitutions are made the resulting analog should be functionally equivalent. Non-conserved substitutions involve replacing one or more amino acids of the amino acid sequence with one or more amino acids which possess dissimilar charge, size, and/or hydrophobicity characteristics.

One or more amino acid insertions may be introduced into the amino acid sequences shown in Figure 2. Amino acid insertions may consist of single amino acid residues or a range of sequential amino acids.

Deletions may consist of the removal of one or more amino acids, or discrete portions from the amino acid sequence shown in Figure 2. The deleted amino acids may or may not be contiguous. The lower limit length of the resulting analog with a deletion mutation is about 10 amino acids, preferably 100 amino acids.

Analogs of a protein as described may be prepared by introducing mutations in the nucleotide sequence encoding the protein. Mutations in nucleotide sequences constructed for expression of analogs of a protein as described must preserve the reading frame of the coding sequences. Furthermore, the mutations will preferably not create complementary regions that could hybridize to produce secondary mRNA structures, such as loops or hairpins, which could adversely affect translation of the receptor mRNA.

Mutations may be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion.

Alternatively, oligonucleotide-directed site specific mutagenesis procedures may be employed to provide an altered gene having particular codons altered according to the substitution, deletion, or insertion required. Deletion or truncation of a protein as described may also be constructed by utilizing convenient restriction endonuclease sites adjacent to the desired deletion. Subsequent to restriction, overhangs may be filled in, and the DNA religated. Exemplary methods of making the alterations set forth above are disclosed by Sambrook et al (Molecular Cloning: A Laboratory Manual, 2nd Ed., Cold Spring Harbor Laboratory Press, 1989).

The proteins as described also include homologs of the amino acid sequence shown in Figure 2 and/or truncations thereof as described herein. Such homologs are proteins whose amino acid sequences are comprised of amino acid sequences whose coding nucleic acid sequences hybridize under stringent hybridization conditions (see discussion of stringent hybridization conditions herein) with a nucleic acid probe used to obtain a protein as described.

A homologous protein includes a protein with an amino acid sequence having at least 70%, preferably 80-90% identity with the amino acid sequence as shown in Figure 2. Homology is typically measured using sequence analysis software (e.g., Sequence Analysis Software Package of the Genetics Computer Group, University of Wisconsin Biotechnology Center, 1710 University Avenue, Madison, WI 53705). Similar amino acid sequences are aligned to obtain the maximum degree of homology (i.e., identity). To this end, it may be necessary to artificially introduce gaps into the sequence. Once the optimal alignment has been set up, the degree of homology (*i.e.,* identity) is established by recording all of the positions in which the amino acids of both sequences are identical, relative to the total number of positions. For example, sequence alignments may be performed using the ALIGN (Trademark) or GENALIGN (Trademark) computer programs (Inteligenetics Suite 5.4, Oxford Molecular). ALIGN uses the Needleman-Wunsch algorithm (Needleman and Wunsch, J. Mol. Biol. 48:443 (1970)) and its later modifications to locate regions of similarity between two sequences. Finding regions of maximum similarity between two sequences can be solved in a rigorous manner using the iterative matrix calculation of the Needleman and Wunsch 1997 algorithm. The analysis is restricted to regions with no internal deletions or insertions, joined by a minimum number of loop-outs or deletions. Sellers (J. Appl. Math (Siam) 26:787 (1974)) developed a true metric measure of the "distance" between sequences and Waterman et al. (Advan. Math 20:367 (1976)) extended this algorithm to include insertions and deletions of arbitrary length. Smith (J. Mol. Biol. 147:195 (1981)) improved the early algorithms to find the subsequences of maximum similarity. The algorithm has been used to analyze sequences as long as 5000 bases by dividing these sequences into segments of 200 to 400 bases, and then reassembling them into a final best match. This method of dividing the sequence and then reassembling it has proven quite robust. The algorithm permits the size of the segment to be specified which the program searches for similarities. The program then assembles the segments after checking overlaps of adjacent subsequences. The weighting of deletions and the relative size of overlaps may be controlled. The program displays the results to show the differences in closely related sequences. GENALIGN is a multiple alignment program. Up to 99 sequences using the Martinez/Regions (Sobel and Martinez, Nucleic Acid Res 14:363 (1985)) or Needleman-Wunsch (Needleman and Wunsch, J. Mol. Biol. 48:443 (1970)) method may be analyzed for alignment. GENALIGN places the sequences in an order that puts the most closely aligned sequence pairs adjacent to each other. A consensus sequence is displayed under the multiple sequence alignments. The sequences used in developing the consensus sequence file for use in other programs. GENALIGN allows the parameters of the search to be changed so that alternate alignments of the sequences can be formed.

These programs are used employing their default settings. The default settings are as follows:

| FastDB | | |
|---|---|---|
| AMINO-Res-length | = | 2 |
| Deletion-weight | = | 5.00 |
| Length-factor | = | 0 |
| Matching-weight | = | 1.00 |
| NUCLEIC-Res-length | = | 4 |
| Spread-factor | = | 50 |
| Findseq | | |

| Search Parameters: | | |
|---|---|---|
| Similarity matrix | | Unitary |
| K-tuple | | 4 |
| Mismatch penalty | | 1 |
| Joining Penalty | | 30 |
| Randomization group length | | 0 |
| Cutoff score | | 5 |
| Alignment Parameters: | | |
| Window size | | 32 |
| Gap penalty | | 1.00 |
| Gap size penalty | | 0.33 |

There is also contemplated isoforms of the proteins as described. An isoform contains the same number and kinds of amino acids as a protein as described, but the isoform has a different molecular structure. The isoforms contemplated herein are those having the same properties as a protein of as described herein.

Polypeptides having a sequence homologous to the sequence of a modified gp100 such as that shown in Figure 2, include naturally-occurring allelic variants, as well as mutants or any other non-naturally occurring variants that are analogous in terms of antigenicity, to a polypeptide having a sequence as shown in Figure 2.

As is known in the art, an allelic variant is an alternate form of a polypeptide that is characterized as having a substitution, deletion, or addition of one or more amino acids that does not alter the biological function of the polypeptide. By "biological function" is meant the function of the polypeptide in the cells in which it naturally occurs, even if the function is not necessary for the growth or survival of the cells. For example, the biological function of a porin is to allow the entry into cells of compounds present in the extracellular medium. The biological function is distinct from the antigenic function. A polypeptide can have more than one biological function.

Allelic variants are very common in nature. For example, a bacterial species (i.e. *C. pneumoniae*) is usually represented by a variety of strains that differ from each other by minor allelic variations. Indeed, a polypeptide that fulfills the same biological function in different strains can have an amino acid sequence that is not identical in each of the strains. Such an allelic variation may be equally reflected at the polynucleotide level.

There is also described a protein as described conjugated with a selected protein, or a selectable marker protein (see below) to produce fusion proteins. Additionally, immunogenic portions and/or fragments of a modified gp100 are described.

An example of fusion polypeptides as described includes a polypeptide or polypeptide derivative as described fused to a polypeptide having adjuvant activity (such as, for example, subunit B of either cholera toxin or *E. coli* heat-labile toxin). Several possibilities exist for achieving fusion. First, the polypeptide as described can be fused to the N-, or preferably, to the C-terminal end of the polypeptide having adjuvant activity. Second, a polypeptide fragment as described can be fused within the amino acid sequence of the polypeptide having adjuvant activity.

The proteins as described (including truncations, analogs, etc.) may be prepared using recombinant DNA methods. Accordingly, the nucleic acid molecules as described having a sequence which encodes a protein of as described may be incorporated in a known manner into an appropriate expression vector which ensures good expression of the protein. Possible expression vectors include but are not limited to cosmids, plasmids, or modified viruses (*e.g.* replication defective retroviruses, adenoviruses and adeno-associated viruses), so long as the vector is compatible with the host cell used. The expression "vectors are suitable for transformation of a host cell" is defined as meaning that the expression vectors contain a nucleic acid molecule as described and attendant regulatory sequences selected on the basis of the host cells to be used for expression, said regulatory sequence being operatively linked to the nucleic acid molecule. "Operatively linked" is intended to mean that the nucleic acid is linked to regulatory sequences in a manner which allows expression of the nucleic acid.

Alos contemplated is a recombinant expression vector as described containing a nucleic acid molecule as described, or a fragment thereof, and the necessary regulatory sequences for the transcription and translation of the inserted nudeotide-sequence. Suitable regulatory sequences may be derived from a variety of sources, including bacterial, fungal, or viral genes. (For example, see the regulatory sequences described in Goeddel, Gene Expression Technology: Methods in Enzymology 185, Academic Press, San Diego, CA (1990)). Selection of appropriate regulatory sequences is dependent on the host cell chosen, and may be readily accomplished by one of ordinary skill in the art. Examples of such regulatory sequences include the following: a transcriptional promoter and enhancer, or RNA polymerase binding sequence, or a ribosomal binding sequence (including a translation initiation signal). Additionally, depending on the host cell chosen and the vector employed, other sequences (such as an origin of replication, additional DNA restriction sites, enhancers, and sequences conferring inducibility of transcription) may be incorporated into the expression vector. It will also be appreciated that the necessary regulatory sequences may also be supplied by the *gp100*/*gp100M* gene and/or its flanking regions in addition to the above mentioned regulatory gene sequence(s).

Also described is a recombinant expression vector comprising a DNA nucleic acid molecule as described cloned into the expression vector in an antisense orientation. That is, the DNA molecule is operatively linked to a regulatory sequence in a manner which allows for expression, by transcription of the DNA molecule, of an RNA molecule which is antisense to a nucleotide sequence comprising the nucleotides as shown in Figure 1. Regulatory sequences operatively linked to the antisense nucleic acid can be chosen which direct the continuous expression of the antisense RNA molecule.

The recombinant expression vectors as described may also contain a selectable marker gene which facilitates the selection of host cells transformed or transfected with a recombinant molecule as described. Examples of selectable marker genes are genes encoding a protein such as G418 and hygromycin (which confer resistance to certain drugs), β-galactosidase, chloramphenicol acetyltransferase, or firefly luciferase. Transcription of the selectable marker gene is monitored by changes in the concentration of the selectable marker protein such as β-galactosidase, chloramphenicol acetyltransferase, or firefly luciferase. If the selectable marker gene encodes a protein conferring antibiotic resistance such as neomycin resistance transformant cells can be selected with G418. As is known to one skilled in the art, cells that have incorporated the selectable marker gene will survive, while cells which do not have any such incorporated detectable marker will die. This makes it possible to visualize and assay for expression of recombinant expression vectors of the invention. It will also be appreciated that selectable markers can be introduced on a separate vector from the nucleic acid of interest.

The recombinant expression vectors may also contain genes which encode a fusion moiety which provides increased expression of the recombinant protein; increased solubility of the recombinant protein; and/or aids in the purification of a target recombinant protein by acting as a ligand in affinity purification. For example, a proteolytic cleavage site may be added to the target recombinant protein to allow separation of the recombinant protein from the fusion moiety subsequent to purification of the fusion protein.

Recombinant expression vectors can be introduced into host cells to produce a transformed host cell. The term "transformed host cell" is intended to include prokaryotic and eukaryotic cells which have been transformed or transfected with a recombinant expression vector of the invention. The terms "transformed with", "transfected with", "transformation" and "transfection" are intended to encompass introduction of nucleic acid (e.g. a vector) into a cell by one of many possible techniques known in the art. Prokaryotic cells can be transformed with nucleic acid by, for example, electroporation or calcium-chloride mediated transformation. Nucleic acid can be introduced into mammalian cells via conventional techniques such as calcium phosphate or calcium chloride co-precipitation, DEAE-dextran-mediated transfection, lipofectin, electroporation or microinjection. Suitable methods for transforming and transfecting host cells can be found in Sambrook et al. (Molecular Cloning: A Laboratory Manual, 2nd Edition, Cold Spring Harbor Laboratory press (1989)), and other laboratory textbooks.

Suitable host cells include a wide variety of prokaryotic and eukaryotic host cells. For example, the proteins as described may be expressed in bacterial cells such as E. *coli,* insect cells (using baculovirus), yeast cells or mammalian cells. Other suitable host cells can be found in Goeddel, Gene Expression Technology: Methods in Enzymology 185, Academic Press, San Diego, CA (1991).

The proteins as described may also be prepared by chemical synthesis using techniques well known in the chemistry of proteins such as solid phase synthesis (Merrifield, J. Am. Chem. Assoc. 85:2149-2154 (1964)) or synthesis in homogenous solution (Houbenweyl, Methods of Organic Chemistry (1987), (ed. E. Wansch) Vol. 15, pts. I and II, Thierne, Stuttgart).

### IV. VACCINES

There is described a vaccine for modulating an animal's immune system wherein the immunogen is an effective amount of a *gp100* or modified gp100, and/or immunogenic fragments thereof, preferably in admixture with a suitable diluent or carrier.

Accordingly, there is described a method of modulating an animal's immune response comprising administering an effective amount of a *gp100* or modified gp100 and/or immunogenic fragments thereof, preferably in admixture with a suitable diluent or carrier, to an animal in need thereof.

The vaccines as described may additionally contain suitable diluents, adjuvants and/or carriers. Preferably, the vaccines contain one or more other adjuvants which can further enhance the immunogenicity of the vaccine *in vivo.* These other one or more adjuvants may be selected from many known adjuvants in the art including, for example, the lipid-A portion of the LPS from gram negative bacteria (endotoxin), trehalose dimycolate of mycobacteria, the phospholipid lysolecithin, dimethyldictadecyl ammonium bromide (DDA), certain linear polyoxypropylene-polyoxyethylene (POP-POE) block polymers, aluminum hydroxide (and other aluminum compounds), and liposomes (see below).

Another preferred adjuvant/immunostimulant is an immunostimulatory oligonucleotide containing unmethylated CpG dinucleotides ("CpG"). CpG is an abbreviation for cytosine-guanosine dinucleotide motifs present in DNA. CpG is known in the art as being an adjuvant when administered by both systemic and mucosal routes (WO 9602555; European Patent EP 468520; Davies et al. (1998) J. Immunol. 160:87; McCluskie and Davis (1998) J. Immunol. 161:4463). In a number of studies, synthetic oligonucleotides derived from BCG gene sequences have also been shown to, be capable of inducing immunostimulatory effects (both in vitro and in vivo; Krieg, (1995) Nature 374:546). Detailed analyses of immunostimulatory oligonucleotide sequences has demonstrated that the CG motif must be in a certain sequence context, and that such sequences are common in bacterial DNA but are rare in vertebrate DNA. (For example, the immunostimulatory sequence is often: purine, purine, C, G, pyrimidine, pyrimidine, wherein the CG motif is not methylated; however other unmethylated CpG sequences are known to be immunostimulatory and as such may also be used in the present invention.)

The vaccines may also include as adjuvants cytokines that are known to enhance immune responses (including GM-CSF, IL-2, IL-12, TNF and IFNγ), co-stimulatory molecules (such of those of the B7 family) and/or other lymphokines. The vaccine may also contain preservatives such as sodium azide, thimersol, beta propiolactone, and binary ethyleneimine.

The vaccine compositions as described are suitable for adminstration to subjects in a biologically compatible form *in vivo*. The expression "biologically compatible form suitable for administration in vivo" as used herein means a form of the substance to be administered in which any toxic effects are outweighed by the therapeutic effects.

The dose of the vaccine may vary according to factors such as the disease state, age, sex, and weight of the , and the ability of the vaccine to elicit a desired response in the animal. Dosage regime may be adjusted to provide the optimum therapeutic response. For example, several divided doses may be administered daily or the dose may be proportionally reduced as indicated by the exigencies of the therapeutic situation. The dose of the vaccine may also be varied to provide optimum preventative dose response depending upon the circumstances.

The vaccines may be administered in a convenient manner such as by injection (subcutaneous, intravenous, intramuscular, intranodal etc.), oral administration, inhalation, transdermal administration (such as topical cream or ointment, etc.), or suppository applications.

As such, there are described (i) a vaccine containing a modified gp100 (or immunogenic fragment thereof) as described; (ii) a composition of matter containing a gp100 (or immunogenic fragment thereof) as described together with a diluent or carrier; (iii) a pharmaceutical composition containing a therapeutically or prophylactically effective amount of a modified gp100 (or immunogenic fragment thereof) as described; (iv) a method for inducing an immune response against a modified gp100 (or immunogenic fragment thereof) in a mammal (for example, a human; alternatively, the method can be used in veterinary application) which involves administering the mammal an immunogenically effective amount of a modified gp100 (or immunogenic fragment thereof) as described to elicit an immune response (for example, a protective or therapeutic immune response to modified gp100); (v) a method for preventing and/or treating melanoma which involves administering a prophylactic or therapeutic amount of a modified gp100 (or immunogenic fragment thereof) as described to an individual in need. Additionally, the invention encompasses the use of a modified gp100 (or immunogenic fragment thereof) as described in the preparation of a medicament for preventing and/or treating of melanoma.

A vaccine of the invention may contain a nucleic acid molecule encoding a modified gp100 protein as described. Such vaccines are refered to as nucleic acid vaccines but are also termed genetic vaccines, polynucleotide vaccines or DNA vaccines, all of which are described. In such case, the modified gp100 protein is produced *in vivo* in the host animal. The vaccines containing nucleic acids may be delivered using a suitable vector (ie. "vaccine vector") including, for example, retroviral vectors, alphaviral, adenoviral vectors, poxviral vectors, other viral vectors, bacterial DNA, plasmids, or free/naked DNA.

Accordingly, there are described (i) a vaccine vector containing a DNA molecule as described placed under the control of elements required for expression; (ii) a composition of matter containing a vaccine vector as described, together with a diluent or carrier; (iii) a pharmaceutical composition containing a therapeutically or prophylactically effective amount of a vaccine vector as described; (iv) a method for inducing an immune response against modified gp100 in a mammal (for example, a human; alternatively, the method can be used in veterinary applications) which involves administering to the mammal an immunogenically effective amount of a vaccine vector as described to elicit an immune response, (for example, a protective or therapeutic immune response to modified gp100); (v) a method for preventing and/or treating melanoma which involves administering a prophylactic or therapeutic amount of a vaccine vector as described to an individual in need. Additionally, the invention encompasses the use of a vaccine vector as described in the preparation of a medicament for preventing and/or treating of melanoma.

### i Vaccine Vector

The vaccine vector may be a poxvirus of other viral vector, bacterial DNA, plasmid or a free/naked DNA. Preferably the vaccine vector is incapable of integration in recipient animal cells. The elements for expression from said vaccine vector may include a promoter suitable for expression in recipient animal cells.

Live vaccine vectors available in the art include viral vectors such as alphaviruses, adenoviruses and poxviruses as well as bacterial vectors (for example, *Shigella, Salmonella, Vibrio cholerae, Lactobacillus,* Bacille Calmette Guérin (BCG), and *Streptococcus*).

An example of an adenovirus vector, as well as a method for constructing an adenovirus vector capable of expressing a DNA molecule of the invention, are described in U.S. Patent No. 4,920,209 (incorporated herein by reference). Poxvirus vectors that can be used include, for example, fowlpox, vaccinia and canary pox virus (as described in U.S. Patent No. 5,766,599 and U.S. Patent No. 5,364,773, U.S. Patent No. 5,756,103, (ALVAC(2), U.S. Patent No. 5,990,091, U.S. Patent No. 6,004,777); Poxvirus vectors capable of expressing a nucleic acid as described can be obtained by homologous recombination as is known to one skilled in the art so that the polynucleotide as described is inserted in the viral genome under appropriate conditions for expression in mammalian cells (see below).

In one preferred aspect the engineered poxvirus vector is ALVAC (which has been derived from canarypox virus). ALVAC does not productively replicate in non-avian hosts, a characteristic thought to improve its safety profile.

ALVAC is an attenuated canarypox virus-based vector that was a plaque-cloned derivative of the licensed canarypox vaccine, Kanapox (Tartaglia et al., Virol. 188:217 (1992)) (U.S. Patent No. 5,756,103). ALVAC has some general properties which are the same as some general properties of Kanapox. ALVAC-based recombinant viruses expressing extrinsic immunogens have also been demonstrated efficacious as vaccine vectors (Tartaglia, J. et al, In AIDS Research Reviews (vol. 3), Koff, W., Wong-Staol, F., Kenedy, R.C. (eds), Marcel Dekker NY, pp. 361-378 (1993); Tartaglia, J. et al. J. Virol. 67:2370 (1993)). For instance, mice immunized with an ALVAC recombinant expressing the rabies virus glycoprotein were protected from lethal challenge with rabies virus (Tartaglia, J. et al. (1992), Virology 188:217) demonstrating the potential for ALVAC as a vaccine vector. ALVAC-based recombinants have also proven efficacious in dogs challenged with canine distemper virus (Taylor, J. et al., (1992), Virology 187:321) and rabies virus (Perkus, M.E. et al., In Combined Vaccines and Simultaneous Administration: Current Issues and Perspective, Annals of New York Academy of Sciences (1994)), in cats challenged with feline leukemia virus (Tartaglia, J. et al. J. Virol. 67:2370 (1993)), and in horses challenged with equine influenza virus (Taylor, J. et al., In Proceedings of the Third International Symposium on Avian Influenza, Univ. of Wisconsin-Madison, Madison, Wisconsin, pp. 331-335 (1993)).

ALVAC (2) is a second-generation ALVAC vector in which vaccinia transcription elements E3L and K3L have been inserted within the C6 locus (U.S. 5,990,091; U.S. 6,004,777). The E3L encodes a protein capable of specifically binding to dsRNA. The K3L ORF has significant homology to E1F-2. Within ALVAC (2) the E3L gene is under the transcriptional control of its natural promoter, whereas K3L has been placed under the control of the early/late vaccine H6 promoter. The E3L and K3L genes act to inhibit PKR activity in cells infected with ALVAC (II), allowing enhancement of the level and persistence of foreign gene expression.

Additional vaccine vector systems involve the use of naturally host-restricted poxviruses. Fowlpox virus (FPV) is the prototypic virus of the Avipox genus of the Poxvirus family. Vectors derived from fowlpox have been generated (i.e., TROVAC, see U.S. Patent No. 5,766,599) Replication of the avipox viruses is limited to avian species (Matthews, Inter Virol. 17:42 (1982)) and there are no reports in the literature of avipoxvirus causing a productive infection in any non-avian species including man. This host restriction provides an inherent safety barrier to transmission of the virus to other species and makes use of avipoxvirus based vaccine vectors in veterinary and human applications an attractive proposition.

FPV has been used advantageously as a vector expressing antigens from poultry pathogens. The hemagglutinin protein of a virulent avian influenza virus was expressed in an FPV recombinant. After inoculation of the recombinant into chickens and turkeys, an immune response was induced which was protective against either a homologous or a heterologous virulent influenza virus challenge (Taylor, J. et al., (1988) Vaccine 6:504). FPV recombinants expressing the surface glycoproteins of Newcastle Disease Virus have also been developed (Taylor, J. et al. (1990) J. Virol. 64:1441; Edbauer, C. et al., (1990) Virology 179:901).

Highly attenuated strain of vaccines, designated MVA, has also been used as a vector for poxvirus-based vaccines. Use of MVA is described in U.S. Patent No. 5,185,146.

Other attenuated poxvirus vectors have been prepared by genetic modifications of wild type strains of virus. The NYVAC vector, for example, is derived by deletion of specific virulence and host-range genes from the Copenhagen strain of vaccinia (Tartaglia, J. et al. (1992) Virology 188:217) and has proven useful as a recombinant vector in eliciting a protective immune response against an expressed foreign antigen (U.S. 5,364,773). The TROVAC vector provides yet another example of an attenuated poxvirus which may be used (U.S. 5,766,599).

Recombinant poxviruses can be constructed in two steps known in the art and analogous to the methods for creating synthetic recombinants of poxviruses such as the vaccinia virus and avipox virus (described in U.S. Patent Nos. 4,769,330; 4,744,848; 4,603,112; 5,100,587; and 5,179,993). Clearly, based on the attenuation profiles of the NYVAC, ALVAC, and TROVAC vectors and their demonstrated ability to elicit both humoral and cellular immunological responses to extrinsic immunogens (Tartaglia, J. et al, In AIDS Research Reviews (vol.3), Koff, W., Wong-Staol, F. and Kenedy, R.C. (eds), Marcel Dekker NY, pp. 361-378 (1993); Tartaglia, J. et al. (1993) J. Virol. 67:2370;; Taylor, J. et al., (1992) Virology 187:321), such recombinant viruses offer a distinct advantage over previously described vaccinia-based recombinant viruses. It can thus be appreciated that provision of a modified gp100 recombinant poxvirus, and of compositions and products therefrom (particularly ALVAC-modified gp100 recombinants and compositions and products therefrom) would be a highly desirable advance over the current state of technology.

Plasmids and/or free/naked nucleic acids (i.e. polynucleotides (DNA or RNA)) as described can also be administered as vaccine vectors to an animal for vaccine (*e.g.,* therapeutic or prophylactic) purpose (US Patent No. 5589466; McDonnell and Askari, NEJM 334:42-45 (1996); Kowalczyk and Ertl, Cell Mol. Life Sci. 55:751-770 (1999)). When a DNA molecule as described is used, it can be in a free/naked or plasmid form. Typically it is a form that is unable to replicate in a mammalian cell and unable to integrate in the mammalian genome. The DNA molecule is also typically placed under the control of a promoter suitable for expression in a mammalian cell. The promoter can function ubiquitously or tissue-specifically. Examples of non-tissue specific promoters include the early Cytomegalovirus (CMV) promoter (described in U.S. Patent No. 4,168,062) and the Rous Sarcoma Virus promoter. The desmin promoter is tissue-specific and drives expression in muscle cells. More generally, useful vectors have been described (*i.e.,* WO 94/21797).

For DNA/RNA vaccination, the polynucleotide as described can encode a precursor or mature form of the modified gp100 or immunogenic fragment thereof. When it encodes a precursor form, the precursor form can be homologous or heterologous. In the latter case, a eucaryotic leader sequence can be used, such as the leader sequence of the tissue-type plasminogen factor (tPA).

Standard techniques of molecular biology for preparing and purifying polynucleotides can be used in the preparation of polynucleotide aspects as described For use as a vaccine, a polynucleotide as described can be formulated according to various methods known to those who are skilled in the art.

First, a polynucleotide can be used in a naked/free form, free of any delivery vehicles (such as anionic liposomes, cationic lipids, microparticles, (*e.g.*, gold microparticles), precipitating agents (*e.g.*, calcium phosphate)) or any other transfection-facilitating agent. In this case the polynucleotide can be simply diluted in a physiologically acceptable solution (such as sterile saline or sterile buffered saline) with or without a carrier. When present, the carrier preferably is isotonic, hypotonic, or weakly hypertonic, and has a relatively low ionic strength (such as provided by a sucrose solution (*e.g.*, a solution containing 20% sucrose))

Alternatively, a polynucleotide can be associated with agents that assist in cellular uptake. It can be, *i.a.,* (i) complemented with a chemical agent that modifies the cellular permeability (such as bupivacaine; see, for example, WO 94/16737), (ii) encapsulated into liposomes, or (iii) associated with cationic lipids or silica, gold, or tungsten microparticles.

Cationic lipids are well known in the art and are commonly used for gene delivery. Such lipids include Lipofectin( also known as DOTMA (N-[1-(2,3-dioleyloxy)propyl]-N,N,N-trimethylammonium chloride), DOTAP (1,2-bis(oleyloxy)-3-(trimethylammonio) propane). DDAB (dimethyldioctadecylammonium bromide), DOGS (dioctadecylamidologlycyl spermine) and cholesterol derivatives such as DC-Chol (3 beta-(N-(N',N'-dimethyl aminomethane)-carbamoyl) cholesterol). A description of these cationic lipids can be found in EP 187,702, WO 90/11092, U.S. Patent No. 5,283,185, WO 91/15501, WO 95/26356, and U.S. Patent No. 5,527,928. Cationic lipids for gene delivery are preferably used in association with a neutral lipid such as DOPE (dioleyl phosphatidylethanolamine), as, for example, described in WO 90/11092.

Other transfection-facilitating compounds can be added to a formulation containing cationic liposomes. A number of them are described in, for example, WO 93/18759, WO 93/19768, WO 94/25608, and WO 95/2397. They include, *i.e.*, spermine derivatives useful for facilitating the transport of DNA through the nuclear membrane (see, for example, WO 93/18759) and membrane-permeabilizing compounds such as GALA, Gramicidine S, and cationic bile salts (see, for example, WO93/19768).

Gold or tungsten microparticles can also be used for gene delivery (as described in WO 91/359 and WO 93/17706). In this case, the microparticle-coated polynucleotides can be injected via intradermal or intraepidermal routes using a needleless injection device ("gene gun"), such as those described, for example, in U.S. Patent No. 4,945,050, U.S. Patent No. 5,015,580, and WO 94/24263.

Anionic and neutral liposomes are also well-known in the art (see, for example, Liposomes: A Practical Approach, RPC New Ed, IRL Press (1990), for a detailed description of methods for making liposomes) and are useful for delivering a large range of products, including polynucleotides.

A vaccine vector as described can also express a cytokine (for example, such as interleukin-2 (IL-2), interleukin-12 (IL-12), granulocyte-macrophage colony stimulating factor (GM-CSF)) and/or co-stimulatory molecules (for example, such as the B7 family of molecules) and/or other lymphokines that enhance the immune response. Thus, a vaccine vector can include an additional DNA sequence encoding, for example, a cytokine and/or lymphokine and/or co-stimulatory molecule placed under the control of suitable elements required for expression in an animal cell.

Alternatively, a composition as described can include several vaccine vectors each being capable of expressing a polypeptide derivative as described, a cytokine and/or lymphokine and/or costimulatory molecules.

### ii Mode of Administration

In vaccination methods for treating or preventing cancer in an animal, a vaccine vector as described can be administered by any conventional route in use in the vaccine field as is known to one skilled in the art. This may include, for example, administration to a mucosal (e.g., ocular, intranasal, oral, gastric, pulmonary, intestinal, rectal, vaginal, or urinary tract) surface, via the parenteral (e.g., subcutaneous, intradermal, intramuscular, intravenous, or intraperitoneal) route or intranodally. Preferred routes depend upon the choice of the vaccine vector. The administration can be achieved in a single dose or repeated at intervals. The appropriate dosage depends on various parameters understood by skilled artisans such as the vaccine vector itself, the route of administration and the condition of the animal to be vaccinated (weight, age and the like).

The administration of the vaccine or immunogen as described may be for either a prophylactic or therapeutic purpose. When provided prophylactically, the immunogen is provided in advance of any evidence or in advance of any symptom due to melanoma, or in patients rendered free of disease by conventional therapies but at significant risk for reoccurrence. The prophylactic administration of the immunogen serves to prevent or attenuate melanoma in a mammal. When provided therapeutically, the immunogen is provided at (or after) the onset of the disease or at the onset of any symptom of the disease. The therapeutic administration of the immunogen serves to attenuate the disease.

A particularly preferred vaccination method encompasses a prime boost protocol. Recent studies have indicated that this protocol, whereby immunization with a poxvirus recombinant expressing a foreign gene product (or other nucleic acid encoding for a gene product) is followed by a boost using a purified subunit preparation form of that gene product (or nucleic acid coding therefor), elicits an enhanced immune response relative to the response elicited with either product alone. Accordingly, it is described to use a modified gp100 in a prime-boost protocol. Examples of methodologies teaching prime-boost protocol are described in WO 98/58956, WO 00/00216, WO 98/56919, WO 97/39771, and WO 98/58956.

The amount of naked/free DNA to be used in a vaccine recipient generally depends on the strength of the promoter used in the DNA construct, the immunogenicity of the expressed gene product, the condition of the animal intended for administration (i.e. the weight, age, and general health of the animal), the mode of administration, and the type of formulation. In general, a therapeutically or prophylactically effective dose from about 1 µg to about 1 mg, preferably, from about 10 µg to about 800 µg and, more preferably, from about 25 µg to about 250 µg, can be administered to human adults. The administration can be achieved in a single dose, repeated at intervals, or incorporated into prime boost protocols (as previously described).

### iii. Oral Vaccines

Non-toxicogenic Vibrio cholerae mutant strains that are useful as a live oral vaccine are described, for example, in US Patent No. 4,882,278 (disclosing a strain in which a substantial amount of the coding sequence of each of the two ctxA alleles has been deleted so that no functional cholerae toxin is produced); WO 92/11354 (strain in which the irgA locus is inactivated by mutation; this mutation can be combined in a single strain with ctxA mutations); and WO 94/1533 (deletion mutant lacking functional ctxA and attRS1 DNA sequences). These strains can be genetically engineered to express heterologous antigens, as described in WO 94/19482. An effective vaccine dose of a Vibrio cholerae strain capable of expressing a polypeptide or polypeptide derivative encoded by a DNA molecule as described can contain, for example, about 1x10⁵ to about 1x10⁹, preferably about 1x10⁶ to about 1x10⁸ viable bacteria in an appropriate volume for the selected route of administration. Preferred routes of administration include all mucosal routes; most preferably, these vectors are administered intranasally or orally.

Attenuated Salmonella typhimurium strains, genetically engineered for recombinant expression of heterologous antigens or not, and their use as oral vaccines are described, for example, in WO 92/11361. Preferred routes of administration include all mucosal routes; most preferably, these vectors are administered intranasally or orally.

As will be readily appreciated by those skilled in the art, other bacterial strains useful as vaccine vectors may also include Shigella flexneri, Streptococcus gordonii, and Bacille Calmette Guerin (as described in WO 88/6626, WO 90/0594, WO 91/13157, WO 92/1796, and WO 92/21376).

In bacterial vectors, a polynucleotide of as described may be inserted into the bacterial genome, can remain in a free state or carried on a plasmid. An adjuvant can also be added to a composition containing a vaccine bacterial vector. A number of adjuvants are known to those skilled in the art. Suitable adjuvants can readily be selected by those skilled in the art.

### V. COMPOSITIONS

A modified gp100 protein and gene, including the gp100M gene (*gp100M*) and gp100M protein as well as the substances identified using the methods described herein, including vaccines, may be formulated into pharmaceutical compositions for adminstration to subjects in a biologically compatible form suitable for administration in vivo. By "biologically compatible form suitable for administration in vivo" is meant a form of the substance to be administered in which any toxic effects are outweighed by the therapeutic effects. The substances may be administered to animals in need thereof. Administration of a therapeutically active amount of the pharmaceutical compositions as described or an "effective amount", are defined as an amount effective, at dosages and for periods of time necessary to achieve the desired result of "modulating an animal's immune system". A therapeutically effective amount of a substance may vary according to factors such as the disease state, age, sex, and weight of the animal, and the ability of immunogen to elicit a desired response in the animal. Dosage regima may be adjusted to provide the optimum therapeutic response. For example, several divided doses may be administered daily or the dose may be proportionally reduced as indicated by the exigencies of the therapeutic situation. "Modulating an animals immune system" is defined as the ability to produce an immune response in a target animal. This response encompasses both cellular and humoral immune responses.

The active substance may be administered in a convenient manner such as by injection (intradermal, intramuscular, subcutaneous, intravenous, intranodal etc.), or by oral administration, inhalation, transdermal application, or rectal administration, or any other route of administration that enables the modulation of an animal's immune system. Depending on the route of administration, the active substance may be coated in a material to protect the compound from the action of enzymes, acids and other natural conditions which may inactivate the compound.

One route of administration which is preferably used is that of intranodal injection of a modified gp100 protein/immunogenic fragment, or nucleic acid encoding said protein/fragment, or any of the compositions as described.

The compositions described herein can be prepared by *per se* known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington's Pharmaceutical Sciences (Remington's Pharmaceutical Sciences (1985), Mack Publishing Company, Easton, Pa., USA) or Handbook of Pharmaceutical Additives (compiled by Michael and Irene Ash, Gower Publishing Limited, Aldershot, England (1995)). On this basis, the compositions include, albeit not exclusively, solutions of the substances in association with one or more pharmaceutically acceptable vehicles or diluents, and may be contained in buffered solutions with a suitable pH and/or be iso-osmotic with physiological fluids. In this regard, reference can be made to U.S. Patent No. 5,843,456.

The utility of the substances, antibodies, and compositions of the invention may be confirmed in experimental model systems.

The following non-limiting examples are illustrative of the present invention:

### EXAMPLES

**ALVAC(2)-gp100M (vCP1584)** is a preparation of recombinant canarypox virus expressing a modified version of human melanoma antigen gp100.

### A. MOLECULAR PROPERTIES (IDENTITIY)

### 1. Recipient characterization:

### a. Parental organism:

Canarypox virus: Family-Poxviridae, Subfamily -Chordopoxviridae, Genus-Avipoxvirus. Canarypox virus is an enveloped virus containing a linear dsDNA genome of approximately 325 kbp. This virus productively replicates exclusively in avian species ((Tripathy D. Avian pox. In: Diseases of Poultry (9th edition: B.W. Calnek et al. Eds.) pp. 583-596).

### b. Description of vector:

ALVAC(2) is a modified, attenuated canarypox virus (U.S. 5,990,091). The original strain of canarypox virus (Rentschler strain) was attenuated by 200 serial passages on primary chick embryo fibroblasts (CEFs). The attenuated virus was plaque isolated and designated ALVAC. To generate ALVAC(2), the ALVAC vector was modified by the insertion of two vaccinia virus coding sequences (E3L and K3L) to enhance the overall efficiency of viral mRNA translation. The E3L-specified gene product is a dsRNA binding protein and the K3L open reading frame (ORF) shares significant sequence similarity to the amino terminal portion of eIF-2( (; Beattie et al. Virology 183:419 (1991); Beattie et al. Virology 210:254 (1995). Both K3L and E3L are capable of inhibiting the activity of a cellular protein kinase (PKR) which, when activated by dsRNA, phosphorylates the translational initiation factor eIF-2a leading to an inhibition of initiation of mRNA translation. Results from several studies have substantiated this proposed mechanism by which the vaccinia K3L and E3L gene products lead to down regulation of PKR activity and enhanced virus-specific gene expression. (Tartaglia, J. et al. 11th Colloque des Cent Gardes, Elsevier Press (1997)).

### c. Derivation of vector from parental organism:

The parental strain of canarypoxvirus (Rentschler strain) was isolated in Germany in 1970, and obtained by Institut Merieux in 1973. The virus was attenuated by 200 serial passages on primary chick embryo fibroblasts (CEFs). The attenuated virus was registered as a vaccine in France in 1975 under the name KANAPOX (ND). The virus was subjected to four successive plaque purifications under agarose and a single plaque isolate was selected and designated ALVAC.

The K3L coding sequence was synthesized by PCR amplification using a plasmid containing Copenhagen vaccinia *Hin*dIII K fragment as template and was placed under the control of the vaccinia H6 promoter. The E3L coding sequence and upstream regulatory element were obtained from a plasmid containing a clone of the *Hin*dIII E fragment from Copenhagen vaccinia. The H6 promoted K3L and the E3L sequences were inserted into a donor plasmid capable of directing their insertion into the C6 site of the parent vector according to the details set out in Example 1. Recombination was performed between the donor plasmid and ALVAC rescuing virus by *in vitro* recombination as previously described (Piccini *et al.,* Methods of Enzymol. 153:545 (1987)). Expression of E3L and K3L in the resultant recombinant virus, vCP1468, was confirmed and vCP1468 was designated ALVAC(2).

### d. Cloning site:

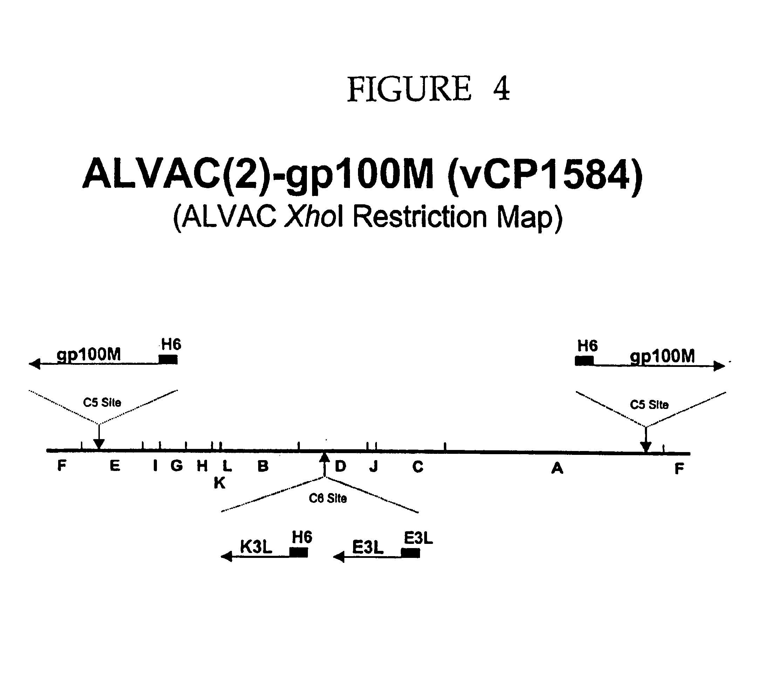

The locus designated C5 was used for the insertion of the modified gp100 coding sequences into the ALVAC(2) vector. By virtue of the C5 locus existing within the extensive inverted terminal repetitions (ITRs) of the virus genome, insertion into this locus results in the occurrence of two copies of the inserted sequence. A schematic of the insertion site is shown in Figure 4. Presently, no function has been ascribed to the C5 encoded polypeptide nor does the deduced amino acid open reading frame encoded in this region share significant homology to any entry in the existing protein databases.

### 2. Donor characterization

### a. Donor organism:

Plasmids pCDNA3-gp100 and PCRII-gp100 each containing the gene encoding human gp100 (melanoma rejection antigen) were used.

### b. Donor genes:

(i) Expressed donor genes: human gp100 melanoma antigen.
(ii) Promoter: Vaccinia virus early/late H6 promoter (PerkusM.E. et al, J. Virol. 63:3829 (1989)).

### c. Donor genes in the recombinant organism:

The coding sequences for a modified human gp100 have been inserted into the ALVAC(2) genome to express the modified gp100 antigen enhanced for the interaction of two CTL epitopes with HLA class I.

### EXAMPLE 1

### Insertion of the vaccinia E3L/K3L coding sequences into the C6 site of ALVAC

The K3L coding sequences were synthesized by PCR amplification using pSD407 (containing Copenhagen vaccinia *Hin*dIII K fragment) as template. The oligonucleotides MPSYN 763 (5'-CCCTCT AGATCG CGATAT CCGTTA AGTTTG TATCGT AATGCT TGCATT TTGTTA TTCGT-3') (SEQ.ID.NO.109) and MPSYN 764 (5'-CCCGAA TTCATA AAAATT ATTGAT GTCTACA-3') (SEQ.ID.NO.110) were used as primers for the PCR reaction. The approximately 325bp PCR fragment was digested with *Xba*I and *Eco*RI yielding a 315bp fragment. This 315bp fragment was purified by isolation from an agarose gel and ligated with *Xba*I and *Eco*RI digested pBSSK+ vector from Stratagene (La Jolla, CA.). The nucleic acid sequence was confirmed. This plasmid was designated pBS 763\764. Digesting pBS 763/764 with *Nru*I and *Xho*I a 340bp fragment was isolated for cloning into the plasmid vector pMM154. PMM154 contains a cassette with the vaccinia H6 promoter controlling an irrelevant gene in the NYVAC tk insertion vector background. PMM154 was prepared by digestion with *Nru*I (partially) and *Xho*I such that the 340bp fragment from pBS 763 / 764 containing the K3L gene could be directionally oriented next to the H6 promoter generating pMPTKH6K3L. The plasmid pMP42GPT containing the dominant selectable marker Eco gpt gene under the control of the Entomopox 42k promoter was digested with *Sma*I and *Bam*HI to yield a 0.7 Kbp 42k- Ecogpt expression cassette. This 0.7 Kbp fragment was purified and ligated into *Sma*I and *Bam*HI cut pMPTKH6K3L generating the plasmid pMPTKH6K3Lgpt. This plasmid was digested with *Xho*I generating a 1.2 Kbp fragment containing the H6/K3L and the 42k/Ecogpt expression cassette which was then gel purified. The 1.2 Kbp *Xho*I fragment was inserted into the *Xho*I site of the ALVAC C6 insertion plasmid pC6L generating pMPC6H6K3Lgpt.

The entire E3L gene is contained within a 2.3 Kbp EcoRI fragment isolated from pSD401VC which contained a clone of the *Hind*III E fragment from Copenhagen vaccinia. The 2.3 Kbp EcoRI fragment was then inserted into pMPC6H6K3Lgpt that had been partially digested with *Eco*RI generating the plasmid pMPC6H6K3E3gpt. The plasmid pMPC6H6K3E3gpt was digested with *Xho*I. The resulting 6.8 Kbp vector fragment was purified and self-ligated, resulting in the plasmid pMPC6E3. The plasmid pMPTKH6K3L was digested with *Psp*AI and the resulting 560bp fragment containing the H6 / K3L expression cassette was ligated into *Psp*AI digested pMPC6E3 resulting in the plasmid construct pMPC6H6K3E3. The plasmid pMPC6H6K3E3 contains the vaccinia H6/K3L expression cassette and the vaccinia E3L gene with the endogenous promoter flanked by the ALVAC C6 insertion site sequences.

### EXAMPLE 2

### Genetic modifications of the donor genes:

Plasmid pCDNA3-gp100 was transformed into MN522 yielding plasmid pMEL gp100 #1. A generic C5 donor plasmid NVQH6C5LSP-18 was digested within the polylinker region with *Bam*HI, treated with alkaline phosphatase and ligated to kinased and annealed oligonucleotides SPC5PL1 (5'-GAT-CGT-CGA-CGA-GCT-CGA-ATT-CG-3') (SEQ.ID.NO.111) and SPC5PL2 (5'-GAT-CCG-AAT-TCG-AGC-TCG-TCG-AC-3') (SEQ.ID.NO.112) generating plasmid NVQH6MC5 #10. Oligonucleotides MELgp01 (5'-CCC-TCG-CGA-TAT-CCG-TTA-AGT-TTG-TAT-CGT-AAT-GGA-TCT-GGT-GCT-AAA-AAG-3') (SEQ.ID.NO.113) and MELgp02 (5'-CCC-CTC-GAG-ATA-AAA-ATC-AGA-CCT-GCT-GCC-CAC-TGA-3') (SEQ.ID.NO.114) were used in PCR with plasmid pMEL gp100 #1 to generate a 2kb fragment containing part of the H6 promoter linked to the 5' end of the gp100 gene. This fragment was digested with EcoRV and *Xho*I and cloned into *Eco*RV/*Xho*I digested NVQH6MC5#10 generating plasmid C5H6MELgp100 #5 which contains the gp100 gene linked to the H6 promoter.

The gp100 gene in plasmid C5H6MELgp100 #5 was sequenced using custom primers. A 65bp deletion was found in this clone and shown to be present in pCDNA3-gp100. Plasmid PCRII-gp100 was used in PCR with oligonucleotides MELgp05(5'-CCC-ATC-TGG-CTC-TTG-GTC-3') (SEQ.ID.NO. 115) and MELgp13 (5'-TGA-CAT-CTC-TGC-CAG-TGT-GGT-3') (SEQ.ID.NO. 116) to generate a 0.6kb fragment. This fragment was digested with *Bam*HI and *Asp*718 and ligated to a 6.5kb *Asp*718/*Bam*HI (partial) fragment from C5H6MELgp100 #5 generating plasmid C5H6MELgp100 which contains the entire gp100 gene under the control of the H6 promoter.

Pre-existing plasmid pC5H6MELgp100 was used as template for site directed mutagenesis of the two CTL epitopes beginning at amino acids 209 and 280, respectively. Primers used were:
209-A
   GCT CAG CCT TCA CCA TTA TGG ACC AGG TGC CTT TCT CC (SEQ.ID.NO.117)
209-B
   GGA GAA AGG CAC CTG GTC CAT AAT GGT GAA GGC TGA CG (SEQ.ID.NO.118)
280-A
   GAG CCT GGC CCA GTC ACT GTT CAG GTG GTC CTG CAG GC (SEQ.ID.NO.119)
280-B
   GCC TGC AGG ACC ACC TGA ACA GTG ACT GGG CCA GGC TC (SEQ.ID.NO.120)

A section containing the modified epitopes was sequenced and isolated as a 440 bp *Nco*1/*Mlu*N1 fragment. This fragment was ligated into pC5H6MELgp100 digested with *Nco*1 and *Mlu*N1, creating a plasmid with the complete gp100 with the modified epitopes 209-2M and 280-9V.

Sequence data revealed a G to C substitution at bp# 10, changing a.a. # 4 from a Valine to a Leucine. This was corrected by PCR using the following primer pair;

MEL25 changes bp# 549 from a C to a G destroying the unique *Nco*1 site for easier screening. It does not change the amino acid.

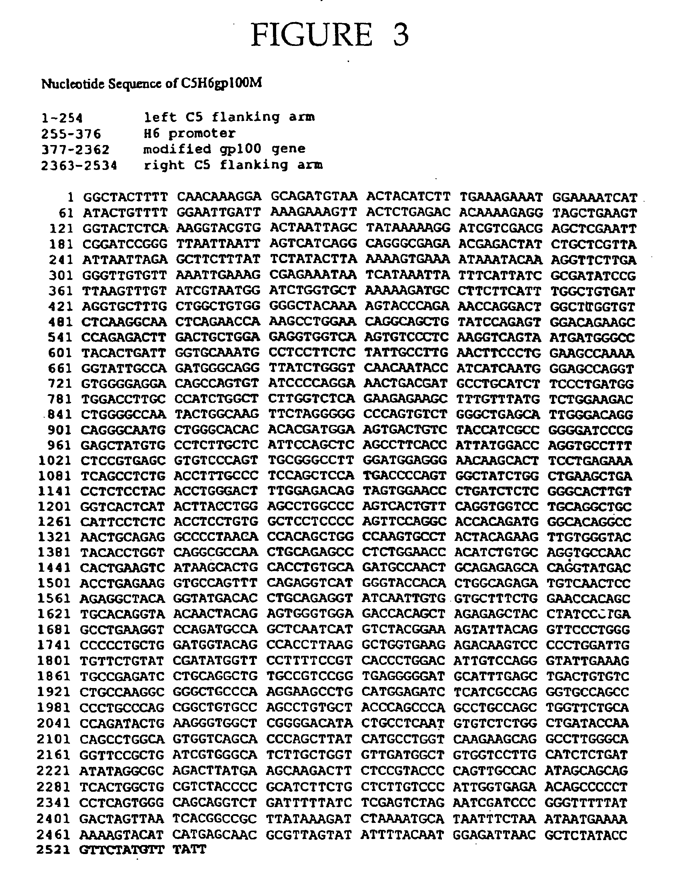

The resulting PCR fragment was digested with *Bam*H1 and *Eco*R5 and replaced the equivalent fragment correcting the error. The resulting plasmid is pC5gp100-M which is shown in Figure 3 (SEQ.ID.NO.123).

### Genetic modification of the recipient:

Recombination between donor plasmid pC5gp100M and ALVAC(2) rescuing virus generated recombinant virus vCP1584, which contains the vaccinia H6 promoted modified human gp100 in the C5 locus.

### EXAMPLE 3

### Screening for the identification and purification of recombinant organisms:

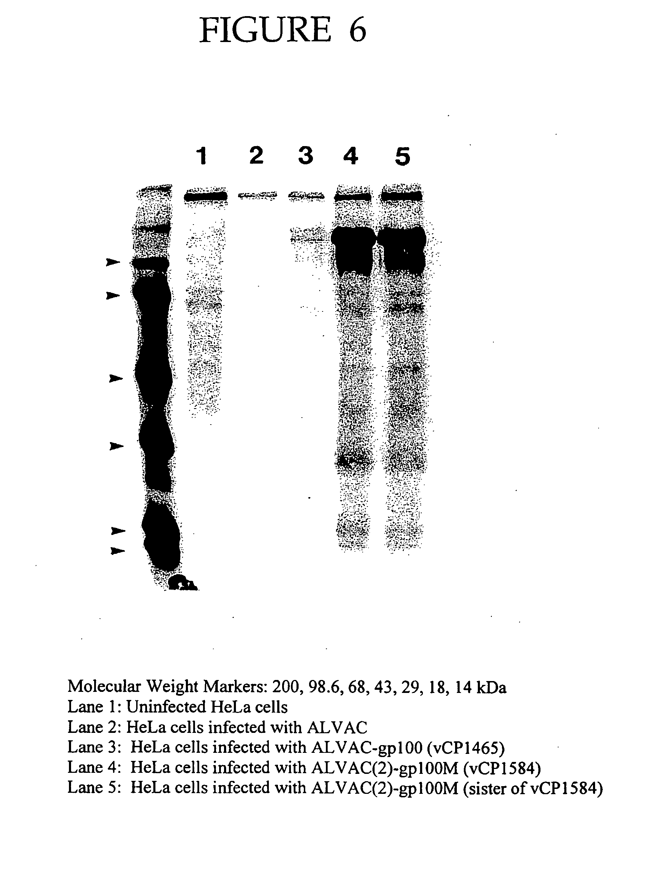

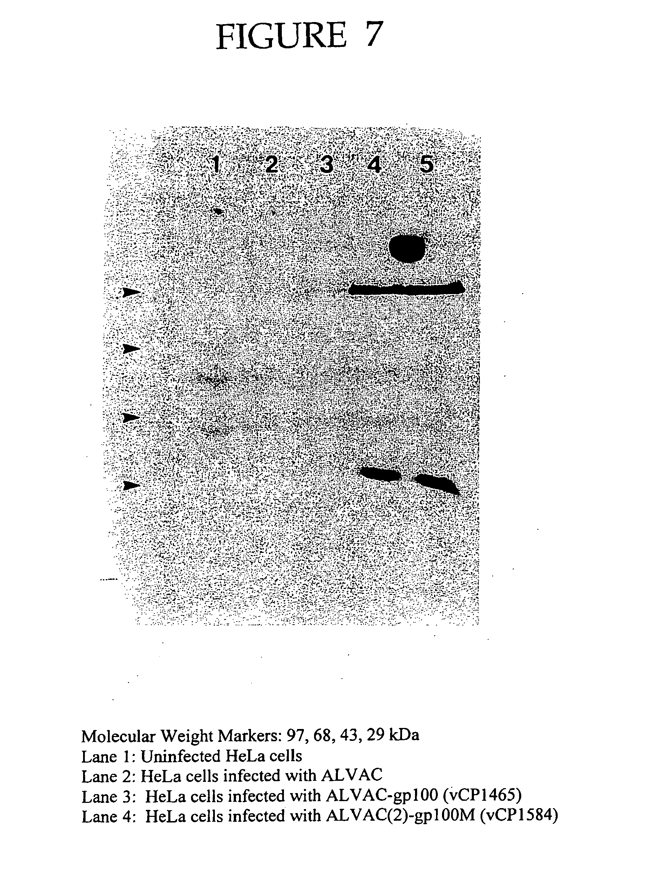

The aspects of screening for the identification and purification of a recombinant organism of the present invention is set out below.
(1) Plaque purification was done using *in situ* plaque hybridization (Piccini et al., Methods of Enzymol. 153:545 (1987)) was used to identify recombinant viruses and to demonstrate purity of final virus preparations. *In situ* plaque hybridization analysis was performed with radiolabelled probes specific for the gp100 construct (a 580 bp fragment) and the C5 insertion locus.
(2) Restriction analysis: Viral genomic DNA was isolated from cells infected with ALVAC parent or ALVAC(2)-gp100M (vCP1584). The genomic DNA was digested with restriction endonucleases *(Hind*III*, Pst* I or *Bam*HI). The resultant DNA fragments were fractionated by electrophoresis through an agarose gel and visualized by ethidium bromide staining. The insertion of the mod gp100 expression cassette at the C5 locus was confirmed.
(3) Immunoprecipitation analyses: These were performed using radiolabeled lysates derived from uninfected HeLa cells or cells infected with either ALVAC parental virus, ALVAC-gp100 (vCP1465) or ALVAC(2)-gp100M (vCP1584) as described previously (Taylor et al . J. Virol. 64:1441 (1990)). Briefly, HeLa cell cultures were infected at an m.o.i. of 10 pfu/cell in methionine-free media supplemented with [35S]-methionine (35uCi/ml). At 18 hrs. post infection, cells were lysed. Immunoprecipitation was performed using a rabbit anti-gp100 serum (AZN-LAM, received from M. Schreurs University of Nijmegen, Netherlands). Immunoprecipitates were fractionated on a 10% SDS-Polyacrylamide gel. The gel was fixed and treated for fluorography with 1M Na-salicylate for 1/2 hr. The dried gel was exposed to Kodak XAR-2 film to visualize the protein species. Results with anti-gp100 demonstrate expression of gp100 in ALVAC-gp100 infected HeLa cells but not for parentally infected cells. (See Figure 6)
(4) Western Blot. HeLa cells were infected for 18 hours at a multiplicity of 10 pfu/cell with ALVAC(2)-gp100M (vCP1584), ALVAC-gp100 (vCP1465) or ALVAC. Cell lysates were separated by SDS-PAGE and transferred to nitrocellulose. The blot was incubated with AZN-LAM (1/5000 dilution) followed by HRP conjugated swine anti-rabbit utilizing the enhanced chemiluminescence (ECL) detection method (Amersham). Results demonstrate expression of full length gp100 in ALVAC-gp100 and ALVAC(2)-gp100M infected cells. (See Figure 7).
(5) Plaque immunoscreen analysis. This was performed on vCP1584 material to determine phenotypic stability of the virus upon passaging. The phenotypic stability of production batch material of ALVAC-gp100M (vCP1584) was analyzed by an immunlogical plaque assay which measures expression of the inserted genes at the plaque level. The assay utilizing permeabilized cells for detection of intracellular as well as surface expression of Hgp100mod was chosen for this test.

Test and control reagents (ALVAC(2)-gp100M (vCP1584) and ALVAC standard and ALVAC-gp100M, respectively) were plated on CEF monolayers under agarose at dilutions resulting in 40-200 plaques per 60 mm dish. 120 hours after incubation at 37°C, the infected monolayers were processed by plaque immunoassay for detection of internal expression of gp100M. Positive and negative plaques were counted for test and control samples. The primary antibody used was Monoclonal Anti-HMB50 at 1:800 dilution. A secondary antibody used was horse radish peroxidase (HRP)-conjugated rabbit anti-mouse antiserum diluted 1:500.

The result of analysis of internal expression of Human modified gp100 by individual plaques produced by (vCP1584) is presented in Table 1.

The result demonstrates that 98.7% of the plaque population of ALVAC-gp100M is expressing gp100M indicating that ALVAC-gp100M is phenotypically stable.

Results of the plaque immunoscreen analysis demonstrate that ALVAC(2)-gp100M is phenotypically stable with respect to expression of gp100. (6) Nucleotide sequence analysis. This was performed on vCP1584 to validate the nucleotide sequence of the H6-promoted melanoma gp100M cassette. The sequence analysis revealed no nucleotide differences relative to the expected sequence, thus no mutations were introducted during the production of vCP1584. In order to carry out this analysis, a pool of plasmid clones containing a 2.2 kb PCR-derived fragment (encompassing the H6-promoted melanoma gp100M cassette), generated from vCP1584 genomic DNA was used.

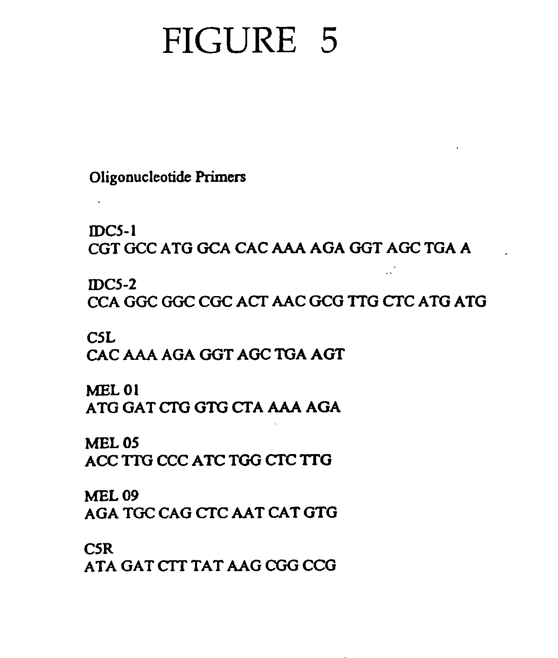

pBS/1584 was generated by pooling 9 positive clones obtained by the ligation of a 2.2 kb PCR fragment (containing the H6-promoted melanoma gp100M cassette from vCP1584), into pBS-sk-(Stratogene). The 2.2 kb PCR fragment was derived from vCP1584 genomic DNA with the oligonucleotide primers, IDC5-1 and IDC5-2 (Figure 5). The nucleotide sequence of the oligonucleotide primers used to sequence pBS/1584 are listed in Figure 5.

### EXAMPLE 4

This example provides results from injection in cynomolgus monkeys of modified gp100 molecules.

### Methods and Experimental Design

### Test System

Cynomolgus monkeys (Macaca fascicularis) purpose bred animals.
Supplier: Siconbrec "Simian Conservation Breeding & Research Center Inc.", Fema Building, 44 Gil Puyat Avenue Makati, Metro Manila, Philippines.
Number of animals in the study: 12 (6 males and 6 females).
Age at initiation of treatment: 26 to 38 months.
- Body weight range at initiation of treatment (day -1):
- males: 1.73 to 2.34 kg
- females: 1.71 to 2.65 kg.

### Animal Husbandry

- Housing: one air-conditioned room;
- temperature: 19 to 25°C (target range),
- relative humidity: >40%
- air changes: minimum 8 air changes per hour,
- lighting cycle: 12 hours light (artificial)/12 hours dark.
- Caging: animals were housed singly in stainless steel mesh cages (approximately 540 x 810 x 760 mm).
- Diet: expanded complete commercial primate diet (Mazuri diet, Special Diet Services Ltd., Witham, Essex, CM8, 3AD, Great Britain) analyzed for chemical and bacterial contaminants.
Quantity distributed: 100g diet/animal/day.
In addition, animals received fruit daily (apple or banana)
Animals were fasted for at least 16 hours before blood sampling for clinical laboratory investigations and before necropsy.
- Water: drinking water *ad libitum* (via bottles).
- Contaminants: no known contaminants were present in diet or water at levels which might have interfered with achieving the objective of the study.

### Pre-Treatment Procedures

- Animal health procedure: all animals received a clinical examination for ill-health on arrival and a veterinary clinical examination during the acclimatization period.
- Acclimatization period: at least 3 weeks between animal arrival and start of treatment.

### Experimental Design

- Allocation to treatment groups was performed during the acclimatization period using a random allocation procedure based on body weight classes.
- Animals were assigned to the treatment groups shown in Table 2. The dose levels administered were shown in Table 3.

### Administration of the Test/Control Articles

### Group 1 and 2 Animals

- Method of administration: injection in the left inguinal lymph node. Animals were lightly anaesthetized before each administration by an intramuscular injection of ketmine hydrochloride (Imalgene® 500 - Merial, Lyon, France). The same lymph node was injected on each occasion (left side). Each injection was followed by a local disinfection with iodine (Vetedine® - Vétoquinol, Lure, France).

### Group 3

- Route: subcutaneous.
- Method of administration: bolus injection using a sterile syringe and needle introduced subcutaneously. Four injection sites were used followed by a local disinfection with iodine (Vétédine® - Vétoquinol, Lure, France). Animals were also lightly anaesthetized before each administration by an intramuscular injection of ketamine hydrochloride (Imalgene® 500 - Merial, Lyon, France) in order to be under the same conditions as groups 1 and 2 animals.
Four injection sites in the dorsal cervical/interscapular regions were used as shown in Table 4.

### ELISPOT Analysis

An ELISPOT assay was used in order to assess the cell mediated immune response generated in the monkeys in the various treatment groups. In particular, an ELISPOT IFNγ assay was used in order to measure IFNγ production from T lymphocytes obtained from the monkeys in response to gp100 antigens.

### Materials and Methods

Plates: MILLIPORE Multiscreen HA plate / MAHA S45.10 (96 wells).
Capture antibodies: MABTECH monoclonal anti-IFNγ antibodies/G-Z4 1 mg/mL.
Detection antibodies: MABTECH monoclonal anti-IFNγ antibodies/7-B6-1-biotin 1 mg/mL.
Enzyme: SIGMA, Extravidin-PA conjuate/E2636
Substrate: BIORAD, NBT/BCIP - Alkaline phosphatase conjugate substrate kit/ref: 170-64 32.

### Coating

Place 100 µL per well of capture antibodies at 1 µg/mL diluted at 1/1000 in carbonate bicarbonate buffer 0.1M pH 9.6 into the multiwell plate. Incubate overnight at 4°C. Wash 4 times in 1X PBS.

### Saturation

Place 200 µL per well of RPMI supplemented with 10% FCS, non essential amino acids, pyruvate, Hepes buffer and Peni-Strepto. Incubate 2 hours at 37°C.

### Test

Cells from the immunized animals are tested against (a) medium alone; (b) pooled peptides at a concentration of 1 mg/mL; and (c) a non specific stimulus (PMA-Iono). The pooled peptides used in this Example to stimulate IFN-γ production were derived from gp100 and are illustrated in Tables 5 to 8. The final volume of each sample is 200 µL. Incubate 20 hours at 37°C.
Wash 4 times in 1X PBS and 0.05% Tween 20.

### Detection

Place 100 µL per well of detection antibodies at 1 µg/mL diluted in 1/1000 1X PBS, 1% BSA and 0.05% Tween 20. Incubate 2 hours at room temperature. Wash 4 times in 1X PBS and 0.05% Tween 20.

### Reaction

Place 100 µL per well of Extravidin-PA conjugate diluted 1/6000 in 1X PBS, 1% BSA and 0.05% Tween 20. Incubate 45 minutes at room temperature. Wash 4 times in 1X PBS and 0.05% Tween 20.

### Substrate Addition

Place 100 µL per well of substrate previously prepared. For example, for 1 plate, prepare: 9.6 mL of distilled water, 0.4 mL of 25X buffer, 0.1 mL of solution A (NBT) and 0.1 mL of solution B (BCIP). Incubate 30-45 minutes at room temperature. Wash in distilled water. Dry and transfer to a plastic film. The number of spots are counted using a Zeiss image analyzer. Each spot corresponds to an individual IFN-γ secreting T cell.

### Results

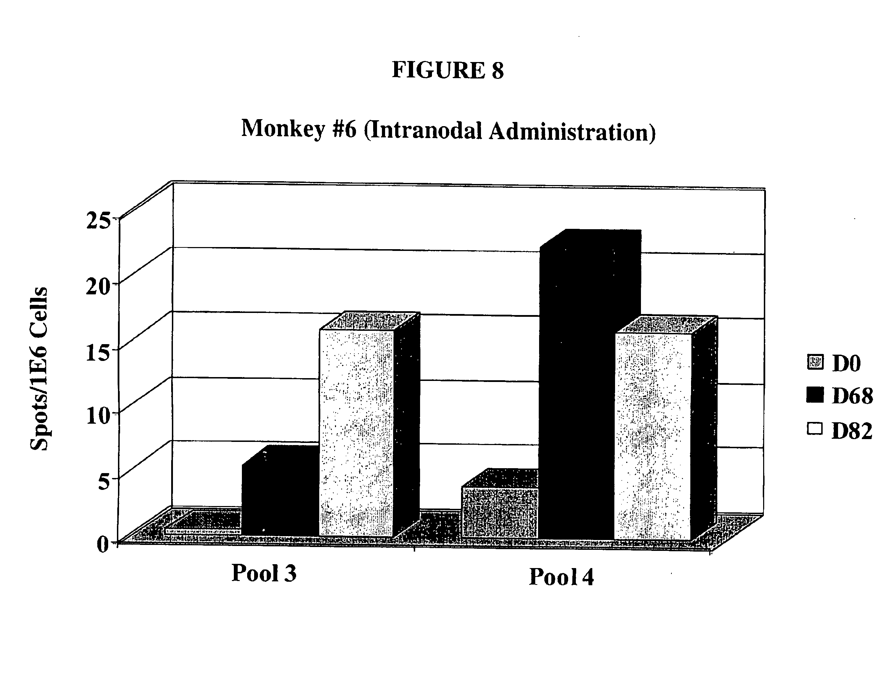

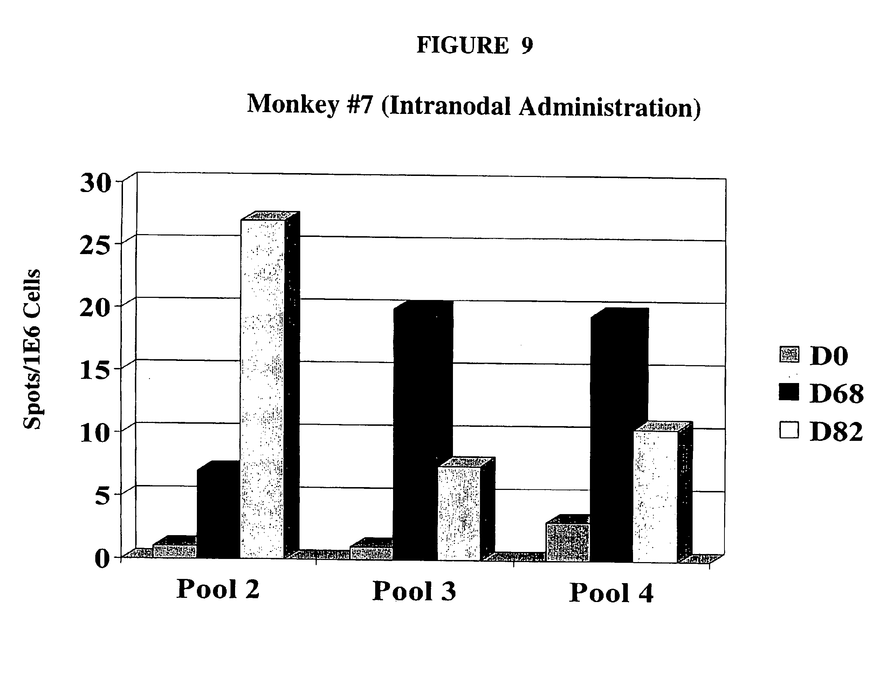

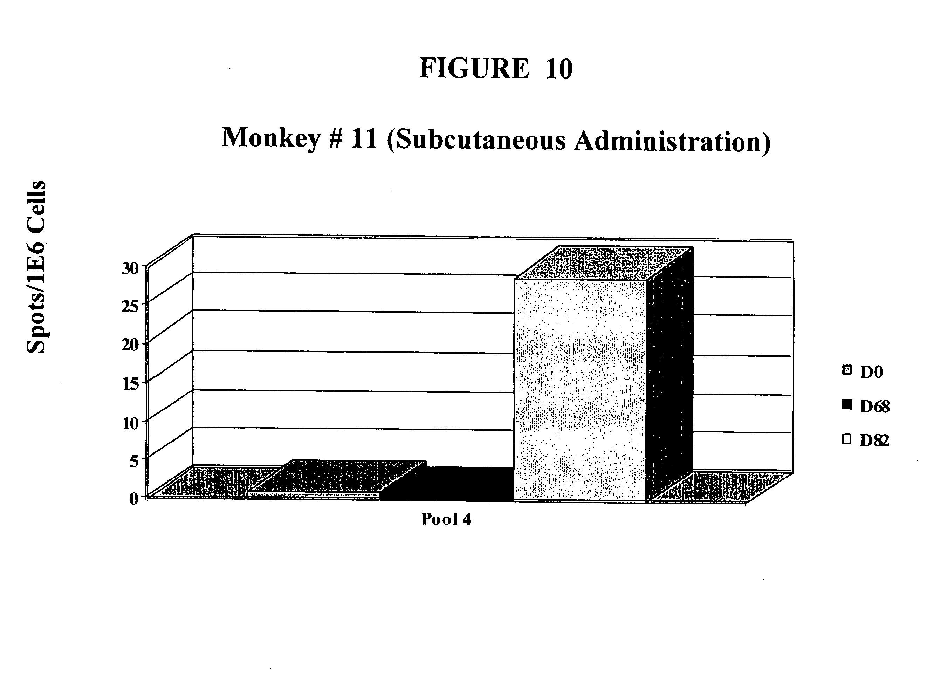

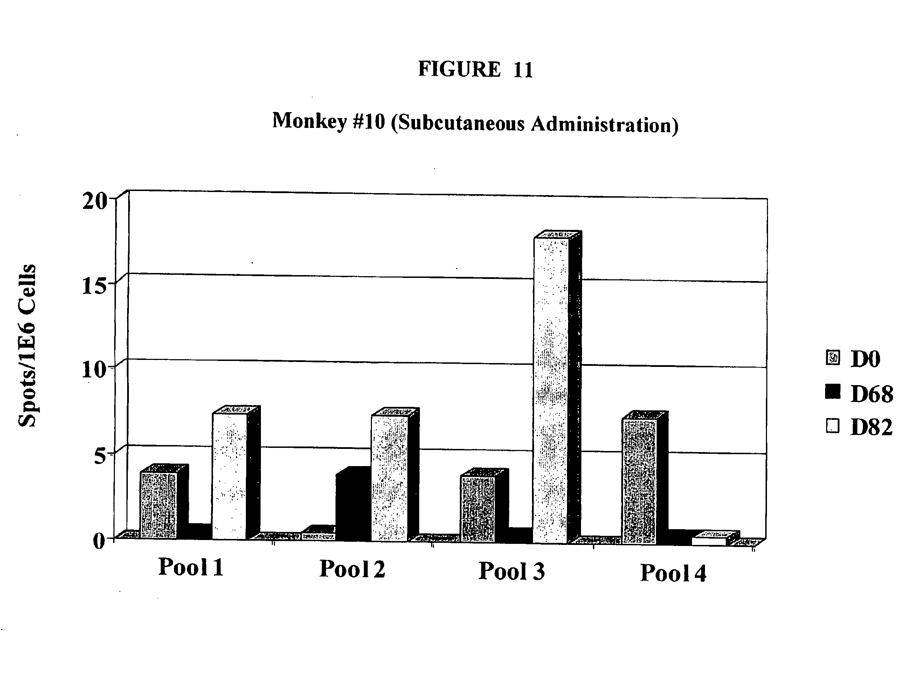

The results of the ELISPOT analysis are shown in Figures 8-11. The results demonstrate that of the animals tested, 2 out of 2 (i.e. 100%) of the animals that received the intranodal administration of the gp100 antigen, and 2 out of 4 (i.e. 50%) of the animals that received the subcutaneous administration of the gp100 antigen had a positive cell mediated immune response.

### ELISA Analysis

The ELISA was performed utilizing standard methodology known in the art. Briefly, the human gp100 ("hgp100"; produced in Baculovirus) was diluted in coating buffer (carbonate-bicarbonate, pH9.6) and added to 96 wells at 0.5ug/well. Plates were placed at 4°C overnight. Plates were then washed and blocking buffer (phosphate buffered saline/0.5% Tween 20/1.0% BSA, pH7.2) was added for 2 hours at 37°C. The plates were then washed and the sera was diluted in dilution buffer (phosphate buffered saline/0.5 % Tween 20/ 0.1 BSA, pH7.2). For this study, monkey sera was diluted to 1:800 and "7" serial 3 fold dilutions were done for each sample tested. The human sera controls were diluted to 1:50 in dilution buffer and "7" serial 2 fold dilutions were performed. Each dilution was done in duplicate. The plates were incubated a further 2 hours at 37°C. The plates were washed and the horse radish peroxidase (HRP)-conjugated anti-human secondary antibody (anti-human Ig whole antibody from sheep (Amersham Life Science, NA933)) diluted 1:100 in dilution buffer was added to the wells and incubated for 1 hour at 37°C. The plates were washed and OPD (o-phenylenediamine dihydrochloride) substrate with H₂O₂ in substrate buffer (50mM phosphate/25mM citrate, pH 7.2) was added to the wells. For a kinetics ELISA, the plate was read repeatedly (2 minute intervals for 15 minutes) unstopped (without "stop" buffer). Plates were read at 450mm.

### Results

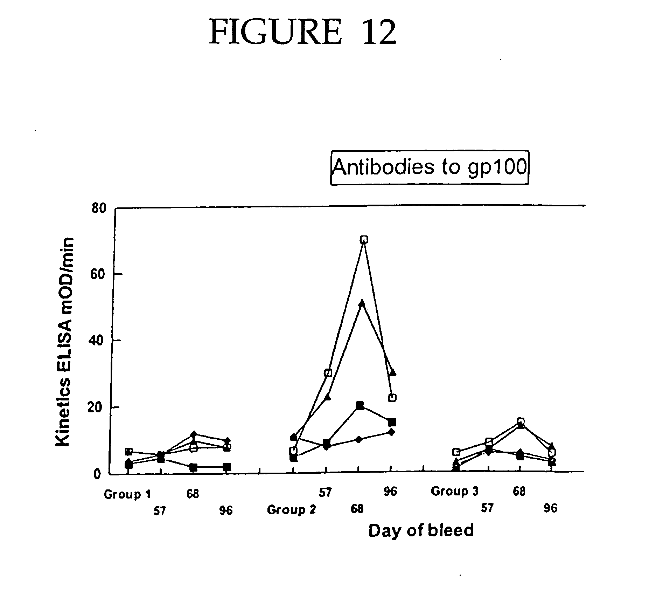

The results of the above experiment are presented in Table 9 and in Figure 12. The animals of group 2 received intranodal injections of ALVAC(2)-gp100(mod) followed by boosts with the modified gp100 peptides 209(2M) and 290(9V); the animals in group 3 received a subcutaneous injection of the ALVAC(2) construct followed by peptide boosts; the animals in group 1 received intranodal injections of saline as a control.

As can be seen from Figure 12, both types of injection of the antigens induced a significant humoral response to the antigen.

In summary, the results of this Example demonstrate that injection of a tumor antigen accoding to the invention induces both a significant humoral and cell mediated response.

### EXAMPLE 5

This example presents data obtained from human melanoma patients primed with ALVAC(2)-gp100M and boosted with modified gp100 peptides (g209-2M and g280-9V).

### Immunization Protocol

5 Patients were immunized subcutaneously in a prime-boost schedule with ALVAC(2)-gp100M ("prime"; lyophilized ALVAC(2)-gp100M resuspended in 1 ml of 0.4% NaCI; 0.5 ml injections (approximately 0.5x10^{7.09} CCID₅₀ per injection)) and peptides g209-2M and g280-9V ("boost"; 1000µg/peptide in 1 ml total volume per week (0.2 ml/injection per day x 5 days)). All patients: 1) were HLA-A0201 positive; 2) were between 18 and 70 years of age; 3) exhibited pathologically confirmed malignant melanoma; 4) demonstrated immunocompetence by reactivity to at least 2 or more out of 7 Cell Mediated Immunity (CMI) skin tests; 5) had blood hematology and chemistry values within the following ranges:

| I) Hematology: | | |
|---|---|---|
| | Hemoglobin | > 100g/L |
| | Granulocytes | > 2.0x10⁹/L |
| | Lymphocytes | > 1.5x10⁹/L |
| | Platelets | > 100x10⁹/L |

| II) Chemistry: | | |
|---|---|---|
| | Serum creatinine | < 150 _mol/L |
| | Serum total bilrubin | <30 _mol/L |
| | AST, ALT, and ALP | Must be < 2x the normal upper |
| | | limit or <5x the normal upper limit |
| | | if due to liver metastases. |

Patients "primed" with ALVAC(2)-gp100M on weeks 1, 4 and 7; "boosted" with peptides on weeks 10 and 13.
ELISPOT Analysis: These results are present in Tables 10 and 11. Peripheral Blood Mononuclear Cells ("PBMNC") were isolated by density centrifugation over Ficoll gradients. Cells were bulk-cultured at 3x10⁶/ml in AIM-V media along with a mixture of g209-2M and g280-9V or the HLA-A*0201 binding Flu peptide (all at 50 µg/ml) for 8 days. IL-2 was added on days 3 and 5 of culture. On day 9, cells were harvested, counted and 2x10⁵ cells/well plus 50 U/ml IL-2, with and without the respective peptides, were plated in nitrocellulose membrane containing ELISPOT plates that had been precoated with anti-INF-γ antibodies. The plates were developed after 48 hours of culture. The numbers reported are the differences between the average of two wells restimulated with peptide and IL-2 and two wells treated only with IL-2.

Responses are the number of spots (counted by the electronic ELISPOT reader but confirmed in most cases by manual counting) per 2x10⁵ PBMNC. The number of CD8+ T cells was not routinely determined but is typically 2-5-fold less than this number.

**TABLE 1**

| Analysis of expression of gp100 antigen by ALVAC-gp100M | | | | |
|---|---|---|---|---|
| | Human gp100M | | | |
| | Positive Plaques | negative plaques | total # of plaques | % positive |
| ALVAC std. | 0 | 571 | 571 | 0 |
| vCP1584 | 387 | 0 | 387 | 100 |
| ALVAC gp100mod L | 875 | 11 | 886 | 98.7 |

**TABLE2**

| **Group Number** | **Route of administration** | **Treatment days and compound administered** | **Number of Animals** |
|---|---|---|---|
| 1 | Intranodal | Saline (NaCl 0.9%): days 28, 42, 56 Then 70, 71, 72, 73, 74 Then 84, 85, 86, 87 and 88 | 4 |
| 2 | Intranodal | ALVAC(2) - gp100 mod: days 28, 42, 56 | 4 |
| | | *mgp100 peptides: days 70, 71, 72, 73, 74 Then 84, 85, 86, 87 and 88 | |
| 3 | Subcutaneous | Saline (NaCl 0.9%): day 1 | 4 |
| | | ALVAC(2) - gp100 mod: days 28, 42, 56 | |
| | | *mgp100 peptides: days 70 and 84 | |

| | | | |
|---|---|---|---|
| *209(2M)-IMDQVPFSY (SEQ.ID.NO.124); 290(9V) YLEPGPVTV (SEQ.ID.NO.125) ■ Group 1 animals (control) received the control article (saline for injection (NaCl 0.9%)). ■ Group 3 animals received the control article (saline for injection (NaCl 0.9%)) on day 1 only. | | | |

**TABLE 3**

| **Group Number** | **Dose level** | **Dose volume (ml/administration)** |
|---|---|---|
| 1 | Saline (NaCl 0.9%): 0 | 0.250 |
| | Dose: 0.25 x 10^{7.4} CCID 50 | |
| 2 | ALVAC (2) - gp100 mod: 0.25 10^{7.4} CCID50 | 0.250 |
| | Dose: 200 µg (Total) of peptides IMDQVPFSY (209(2M)), and YLEPGPVTV (290(9V)) (100µg each) | 0.2 |
| 3 | Saline (NaCl 0.9%) | 0.250 |
| | ALVAC(2) - gp100 mod: 0.25 10^{7.4} CCID 50 | 0.250 |
| | Dose: 200 µg (Total) of peptides IMDQVPFSY (209(2M)), and YLEPGPVTV (290(9V)) (100µg each) | 0.2 |

**TABLE 4**

| **Days** | **Sites used** |
|---|---|
| 1 and 28 | lower left |
| 42 | upper left |
| 56 | upper right |
| 70 | lower left |
| 84 | lower right |

**TABLE 5**

| Peptide Pool #1 | | |
|---|---|---|
| Peptide | Sequence | SEQ.ID.NO. |
| 1329 | HLAVIGALLAVGATK | SEQ.ID.NO.3 |
| 1330 | GALLAVGATKVPRNQ | SEQ.ID.NO.4 |
| 1331 | VGATKVPRNQDWLGV | SEQ.ID.NO.5 |
| 1332 | VPRNQDWLGVSRQLR | SEQ.ID.NO.6 |
| 1333 | DWLGVSRQLRTKAWN | SEQ.ID.NO.7 |
| 1334 | SRQLRTKAWNRQLYP | SEQ.ID.NO.8 |
| 1335 | TKAWNRQLYPEWTEA | SEQ.ID.NO.9 |
| 1336 | RQLYPEWTEAQRLDC | SEQ.ID.NO.10 |
| 1337 | EWTEAQRLDCWRGGQ | SEQ.ID.NO.11 |
| 1338 | QRLDCWRGGQVSLKV | SEQ.ID.NO.12 |
| 1339 | WRGGQVSLKVSNDGP | SEQ.ID.NO.13 |
| 1340 | VSLKVSNDGPTLIGA | SEQ.ID.NO.14 |
| 1344 | IALNFPGSQKVLPDG | SEQ.ID.NO.15 |
| 1345 | PGSQKVLPDGQVIWV | SEQ.ID.NO.16 |
| 1346 | VLPDGQVIWVNNTII | SEQ.ID.NO.17 |
| 1347 | QVIWVNNTIINGSQV | SEQ.ID.NO.18 |
| 1348 | NNTIINGSQVWGGQP | SEQ.ID.NO.19 |
| 1349 | NGSQVWGGQPVYPQE | SEQ.ID.NO.20 |
| 1350 | WGGQPVYPQETDDAC | SEQ.ID.NO.21 |
| 1351 | VYPQETDDACIFPDG | SEQ.ID.NO.22 |
| 1352 | TDDACIFPDGGPCPS | SEQ.ID.NO.23 |
| 1353 | IFPDGGPCPSGSWSQ | SEQ.ID.NO.24 |
| 1355 | GSWSQKRSFVYVWKT | SEQ.ID.NO.25 |
| 1356 | KRSFVYVWKTWGQYW | SEQ.ID.NO.26 |
| 1357 | YVWKTWGQYWQVLGG | SEQ.ID.NO.27 |
| 1358 | WGQYWQVLGGPVSGL | SEQ.ID.NO.28 |
| 1359 | QVLGGPVSGLSIGTG | SEQ.ID.NO.29 |

**TABLE 6**

| Peptide Pool #2 | | |
|---|---|---|
| Peptide | Sequence | SEQ.ID.NO. |
| 1360 | PVSGLSIGTGRAMLG | SEQ.ID.NO.30 |
| 1361 | SIGTGRAMLGTHTME | SEQ.ID.NO.31 |
| 1362 | RAMLGTHTMEVTVYH | SEQ.ID.NO.32 |
| 1363 | THTMEVTVYHRRGSR | SEQ.ID.NO.33 |
| 1364 | VTVYHRRGSRSYVPL | SEQ.ID.NO.34 |
| 1365 | RRGSRSYVPLAHSSS | SEQ.ID.NO.35 |
| 1366 | SYVPLAHSSSAFTIT | SEQ.ID.NO.36 |
| 1368 | AFTITDQVPFSVSVS | SEQ.ID.NO.37 |
| 1369 | DQVPFSVSVSQLRAL | SEQ.ID.NO.38 |
| 1370 | SVSVSQLRALDGGNK | SEQ.ID.NO.39 |
| 1372 | DGGNKHFLRNQPLTF | SEQ.ID.NO.40 |
| 1373 | HFLRNQPLTFALQLH | SEQ.ID.NO.41 |
| 1374 | QPLTFALQLHDPSGY | SEQ.ID.NO.42 |
| 1375 | ALQLHDPSGYLAEAD | SEQ.ID.NO.43 |
| 1379 | DFGDSSGTLISRALV | SEQ.ID.NO.44 |
| 1380 | STGLISRALVVTHTY | SEQ.ID.NO.45 |
| 1381 | SRALVVTHTYLEPGP | SEQ.ID.NO.46 |
| 1382 | VTHTYLEPGPVTAQV | SEQ.ID.N0.47 |
| 1383 | LEPGPVTAQVVLQAA | SEQ.ID.NO.48 |
| 1384 | VTAQVVLQAAIPLTS | SEQ.ID.NO.49 |
| 1385 | VLQAAIPLTSCGSSP | SEQ.ID.NO.50 |
| 1386 | IPLTSCGSSPVPGTT | SEQ.ID.NO.51 |
| 1388 | VPGTTDGHRPTAEAP | SEQ.ID.NO.52 |
| 1389 | DGHRPTAEAPNTTAG | SEQ.ID.NO.53 |
| 1390 | TAEAPNTTAGQVPTT | SEQ.ID.NO.54 |
| 1392 | QVPTTEVVGTTPGQA | SEQ.ID.NO.55 |
| 1393 | EVVGTTPGQAPTAEP | SEQ.ID.NO.56 |

**TABLE 7**

| Peptide Pool #3 | | |
|---|---|---|
| Peptide | Sequence | SEQ.ID.NO. |
| 1394 | TPGQAPTAEPSGTTS | SEQ.ID.NO.57 |
| 1395 | PTAEPSGTTSVQVPT | SEQ.ID.NO.58 |
| 1396 | SGTTSVQVPTTEVIS | SEQ.ID.NO.59 |
| 1397 | VQVPTTEVISTAPVQ | SEQ.ID.NO.60 |
| 1398 | TEVISTAPVQMPTAE | SEQ.ID.NO.61 |
| 1399 | TAPVQMPTAESTGMT | SEQ.ID.NO.62 |
| 1400 | MPTAESTGMTPEKVP | SEQ.ID.NO.63 |
| 1401 | STGMTPEKVPVSEVM | SEQ.ID.NO.64 |
| 1402 | PEKVPVSEVMGTTLA | SEQ.ID.NO.65 |
| 1403 | VSEVMGTTLAEMSTP | SEQ.ID.NO.66 |
| 1404 | GTTLAEMSTPEATGM | SEQ.ID.NO.67 |
| 1405 | EMSTPEATGMTPAEV | SEQ.ID.NO.68 |
| 1408 | SIVVLSGTTAAQVTT | SEQ.ID.NO.69 |
| 1409 | SGTTAAQVTTTEWVE | SEQ.ID.NO.70 |
| 1410 | AQVTTTEWVETTARE | SEQ.ID.NO.71 |
| 1411 | TEWVETTARELPIPE | SEQ.ID.NO.72 |
| 1412 | TTARELPIPEPEGPD | SEQ.ID.NO.73 |
| 1413 | LPIPEPEGPDASSIM | SEQ.ID.NO.74 |
| 1414 | PEGPDASSIMSTESI | SEQ.ID.NO.75 |
| 1415 | ASSIMSTESITGSLG | SEQ.ID.NO.76 |
| 1416 | STESITGSLGPLLDG | SEQ.ID.NO.77 |
| 1417 | TGSLGPLLDGTATLR | SEQ.ID.NO.78 |
| 1418 | PLLDGTATLRLVKRQ | SEQ.ID.NO.79 |
| 1419 | TATLRLV K RQV PLDC | SEQ.ID.NO.80 |
| 1420 | LVKRQVPLDCVLYRY | SEQ.ID.NO.81 |
| 1421 | VPLDCVLYRYGSFSV | SEQ.ID.NO.82 |
| 1422 | VLYRYGSFSVTLDIV | SEQ.ID.NO.83 |

**Table 8**

| Peptide Pool #4 | | |
|---|---|---|
| Peptide | Sequence | SEQ.ID.NO. |
| 1424 | TLDIVQGIESAEILQ | SEQ.ID.NO.84 |
| 1425 | QGIESAEILQAVPSG | SEQ.ID.NO.85 |
| 1426 | AEILQAVPSGEGDAF | SEQ.ID.NO.86 |
| 1427 | AVPSGEGDAFELTVS | SEQ.ID.NO.87 |
| 1428 | EGDAFELTVSCQGGL | SEQ.ID.NO.88 |
| 1429 | ELTVSCQGGLPKEAC | SEQ.ID.NO.89 |
| 1430 | CQGGLPKEACMEISS | SEQ.ID.NO.90 |
| 1431 | PKEACMEISSPGCQP | SEQ.ID.NO.91 |
| 1432 | MEISSPGCQPPAQRL | SEQ.ID.NO.92 |
| 1434 | PAQRLCQPVLPSPAC | SEQ.ID.NO.93 |
| 1435 | CQPVLPSPACQLVLH | SEQ.ID.NO.94 |
| 1436 | PSPACQLVLHQILKG | SEQ.ID.NO.95 |
| 1437 | QLVLHQILKGGSGTY | SEQ.ID.NO.96 |
| 1441 | LADTNSLAVVSTQLI | SEQ.ID.NO.97 |
| 1442 | SLAVVSTQLIMPGQE | SEQ.ID.NO.98 |
| 1443 | STQLIMPGQEAGLGQ | SEQ.ID.NO.99 |
| 1444 | MPGQEAGLGQVPLIV | SEQ.ID.NO.100 |
| 1445 | AGLGQVPLIVGILLV | SEQ.ID.NO.101 |
| 1448 | LMAVVLASLIYRRRL | SEQ.ID.NO.102 |
| 1450 | YRRRLMKQDFSVPQL | SEQ.ID.NO.103 |
| 1451 | MKQDFSVPQLPHSSS | SEQ.ID.NO.104 |
| 1452 | SVPQLPHSSSHWLRL | SEQ.ID.NO.105 |
| 1453 | PHSSSHWLRLPRIFC | SEQ.ID.NO.106 |
| 1454 | HWLRLPRIFCSCPIG | SEQ.ID.NO.107 |
| 1455 | PRIFCSCPIGENSPL | SEQ.ID.NO.108 |

**TABLE 9**

| | DAY (mOD/min) | | | |
|---|---|---|---|---|
| Monkey # | 0 | 57 | 68 | 96 |
| 1 | 3 | 5 | 2 | 2 |
| 2 | 4 | 6 | 12 | 10 |
| 3 | 7 | 6 | 10 | 8 |
| 4 | 7 | 6 | 8 | 8 |
| 5 | 5 | 9 | 20 | 15 |
| 6 | 11 | 8 | 10 | 12 |
| 7 | 11 | 23 | 51 | 30 |
| 8 | 7 | 30 | 70 | 22 |
| 9 | 1 | 7 | 5 | 3 |
| 10 | 2 | 6 | 6 | 4 |
| 11 | 3 | 7 | 14 | 8 |
| 12 | 6 | 9 | 15 | 6 |

**TABLE 10**

| **Gp100-specific responses to g209-2M and g280-9V*** | | | | | | |
|---|---|---|---|---|---|---|
| **Patient** | **Pre 1^{st} Injection** | **Pre 2^{nd} Injection** | **Pre 3^{rd} Injection** | **Pre 4^{th} Injection** | **Pre 5^{th} Injection** | **4 wks post vaccination** |
| #1 | 0 | 0 | 0 | ND | ND | 2±1.4 |
| #2 | 0 | 14±2.8 | 54±6.4 | 16±7.8 | ND | ND |
| #3 | 0 | 0 | ND | ND | ND | ND |
| #4 | 0 | 0 | 24±13.4 | 1±2.1 | ND | ND |
| #5 | ND | 6±6.4 | ND | ND | ND | ND |

**TABLE 11**

| **Flu-peptide specific responses*** | | | | | | |
|---|---|---|---|---|---|---|
| **Patient** | **Pre 1^{st} Injection** | **Pre 2^{nd} Injection** | **Pre 3^{rd} Injection** | **Pre 4^{th} Injection** | **Pre 5^{th} Injection** | **4 wks post vaccination** |
| #1 | >150 | ND | >70 | ND | ND | 12.5 |
| #2 | ND | 0 | 24 | 0 | ND | ND |
| #3 | 23.5 | 7 | ND | ND | ND | ND |
| #4 | 0 | 29 | 13.5 | 11.5 | ND | ND |
| #5 | ND | >200 | ND | ND | ND | ND |

| | | | | | | |
|---|---|---|---|---|---|---|
| * ND signifies that the values were not determined for the sample. | | | | | | |

## Claims

1. The use of (i) a poxvirus vector which has inserted therein a nucleic acid sequence as shown in Figure 1 (SEQ ID NO: 1) and (ii) peptides consisting of amino acid sequences according to SEQ ID NO: 124 and SEQ ID NO: 125, in the manufacture of a medicament for producing an immunogenic response to human melanoma by sequential administration.

2. The use as claimed in claim 1 wherein the poxvirus vector is ALVAC.

3. The use as claimed in claim 1 wherein the poxvirus vector is ALVAC-2.

4. Pharmaceutical composition comprising a poxvirus vector which has inserted therein a nucleic acid sequence as shown in Figure 1 (SEQ ID NO: 1) for priming and the peptide of SEQ ID NO: 124, and the peptide of SEQ ID NO: 125 for boosting; wherein the composition is adapted for separate administration of the vector and peptides.

5. A composition as claimed in claim 4 wherein the poxvirus vector is ALVAC.

6. A composition as claimed in claim 4 wherein the poxvirus vector is ALVAC-2.

## Patentansprüche

1. Verwendung (i) eines Poxvirusvektors, der darin inseriert eine in Figur 1 (SEQ ID NR: 1) gezeigte Nukleinsäuresequenz aufweist, und (ii) Peptide, bestehend aus den Aminosäuresequenzen gemäß SEQ ID NR: 124 und SEQ ID NR: 125, zur Herstellung eines Medikaments für die Erzeugung einer Immunantwort gegen humanes Melanom durch sequentielle Verabreichung.

2. Verwendung nach Anspruch 1, wobei der Poxvirusvektor ALVAC ist.

3. Verwendung nach Anspruch 1, wobei der Poxvirusvektor ALVAC-2 ist.

4. Pharmazeutische Zusammensetzung, umfassend einen Poxvirusvektor, der darin inseriert eine in Figur 1 (SEQ ID NR: 1) gezeigte Nukleinsäuresequenz zum Primen aufweist, und das Peptid der SEQ ID NR: 124 und das Peptid der SEQ ID NR: 125 zum Boosten, wobei die Zusammensetzung für die getrennte Verabreichung des Vektors und der Peptide hergerichtet ist.

5. Zusammensetzung nach Anspruch 4, wobei der Poxvirusvektor ALVAC ist.

6. Zusammensetzung nach Anspruch 4, wobei der Poxvirusvektor ALVAC-2 ist.

## Revendications

1. Utilisation (i) d'un vecteur poxviral, dans lequel une séquence d'acide nucléique telle que représentée sur la figure 1 (SEQ ID NO : 1) est insérée et (ii) de peptides constitués des séquences d'acides aminés selon SEQ ID NO : 124 et SEQ ID NO : 125, dans la fabrication d'un médicament destiné à la production d'une réponse immunogène au mélanome humain par administration séquentielle.

2. Utilisation selon la revendication 1, dans laquelle vecteur poxviral est l'ALVAC.

3. Utilisation selon la revendication 1, dans laquelle vecteur poxviral est l'ALVAC-2.

4. Composition pharmaceutique comprenant un vecteur poxviral, dans lequel une séquence d'acide nucléique telle que représentée sur la figure 1 (SEQ ID NO : 1) est insérée pour la primo-vaccination et le peptide de séquence SEQ ID NO : 124 et le peptide de séquence SEQ ID NO : 125 pour le rappel ; où la composition est adaptée à une administration séparée du vecteur et des peptides.

5. Composition selon la revendication 4, dans laquelle le vecteur poxviral est l'ALVAC.

6. Composition selon la revendication 4, dans laquelle le vecteur poxviral est l'ALVAC-2.


### SEQUENCE LISTING

<110> Aventis Pasteur Limited
   Barber, Brian
   Berinstein, Neil
   Moingeon, Philippe
   Tartaglia, James
   Tine, John
<120> Modified GP100 and Uses Thereof
<130> 11014-13
<140> PCT/CA00/01254
   <141> 2000-10-20
<150> US 60/160,879
   <151> 1999-10-22
<150> US 60/223,325
   <151> 2000-08-07
<160> 125
<170> PatentIn version 3.0
<210> 1
   <211> 1986
<212> DNA
   <213> Artificial
<220>
   <223> modified gp 100
<400> 1
<210> 2
   <211> 661
   <212> PRT
   <213> Artificial
<220>
   <223> modified gp 100
   <400> 2
   Met Asp Leu Val Leu Lys Arg Cys Leu Leu His Leu Ala Val Ile Gly
   1               5                   10                  15
   Ala Leu Leu Ala Val Gly Ala Thr Lys Val Pro Arg Asn Gln Asp Trp
               20                  25                  30
   Leu Gly Val Ser Arg Gln Leu Arg Thr Lys Ala Trp Asn Arg Gln Leu
           35                  40                  45
   Tyr Pro Glu Trp Thr Glu Ala Gln Arg Leu Asp Cys Trp Arg Gly Gly
       50                  55                  60
   Gln Val Ser Leu Lys Val Ser Asn Asp Gly Pro Thr Leu Ile Gly Ala
   65                  70                  75                  80
   Asn Ala Ser Phe Ser Ile Ala Leu Asn Phe Pro Gly Ser Gln Lys Val
   85 90 95
   Leu Pro Asp Gly Gln Val Ile Trp Val Asn Asn Thr Ile Ile Asn Gly
   100 105 110
   Ser Gln Val Trp Gly Gly Gln Pro Val Tyr Pro Gln Glu Thr Asp Asp
   115 120 125
   Ala Cys Ile Phe Pro Asp Gly Gly Pro Cys Pro Ser Gly Ser Trp Ser
   130 135 140
   Gln Lys Arg Ser Phe Val Tyr Val Trp Lys Thr Trp Gly Gln Tyr Trp
   145 150 155 160
   Gln Val Leu Gly Gly Pro Val Ser Gly Leu Ser Ile Gly Thr Gly Arg
   165 170 175
   Ala Met Leu Gly Thr His Thr Met Glu Val Thr Val Tyr His Arg Arg
   180 185 190
   Gly Ser Arg Ser Tyr Val Pro Leu Ala His Ser Ser Ser Ala Phe Thr
   195 200 205
   Ile Met Asp Gln Val Pro Phe Ser Val Ser Val Ser Gln Leu Arg Ala
   210 215 220
   Leu Asp Gly Gly Asn Lys His Phe Leu Arg Asn Gln Pro Leu Thr Phe
   225 230 235 240
   Ala Leu Gln Leu His Asp Pro Ser Gly Tyr Leu Ala Glu Ala Asp Leu
   245 250 255
   Ser Tyr Thr Trp Asp Phe Gly Asp Ser Ser Gly Thr Leu Ile Ser Arg
   260 265 270
   Ala Leu Val Val Thr His Thr Tyr Leu Glu Pro Gly Pro Val Thr Val
   275 280 285
   Gln Val Val Leu Gln Ala Ala Ile Pro Leu Thr Ser Cys Gly Ser Ser
   290 295 300
   Pro Val Pro Gly Thr Thr Asp Gly His Arg Pro Thr Ala Glu Ala Pro
   305 310 315 320
   Asn Thr Thr Ala Gly Gln Val Pro Thr Thr Glu Val Val Gly Thr Thr
   325 330 335
   Pro Gly Gln Ala Pro Thr Ala Glu Pro Ser Gly Thr Thr Ser Val Gln
   340 345 350
   Val Pro Thr Thr Glu Val Ile Ser Thr Ala Pro Val Gln Met Pro Thr
   355 360 365
   Ala Glu Ser Thr Gly Met Thr Pro Glu Lys Val Pro Val Ser Glu Val
   370 375 380
   Met Gly Thr Thr Leu Ala Glu Met Ser Thr Pro Glu Ala Thr Gly Met
   385 390 395 400
   Thr Pro Ala Glu Val Ser Ile Val Val Leu Ser Gly Thr Thr Ala Ala
   405 410 415
   Gln Val Thr Thr Thr Glu Trp Val Glu Thr Thr Ala Arg Glu Leu Pro
   420 425 430
   Ile Pro Glu Pro Glu Gly Pro Asp Ala Ser Ser Ile Met Ser Thr Glu
   435 440 445
   Ser Ile Thr Gly Ser Leu Gly Pro Leu Leu Asp Gly Thr Ala Thr Leu
   450 455 460
   Arg Leu Val Lys Arg Gln Val Pro Leu Asp Cys Val Leu Tyr Arg Tyr
   465 470 475 480
   Gly Ser Phe Ser Val Thr Leu Asp Ile Val Gln Gly Ile Glu Ser Ala
   485 490 495
   Glu Ile Leu Gln Ala Val Pro Ser Gly Glu Gly Asp Ala Phe Glu Leu
   500 505 510
   Thr Val Ser Cys Gln Gly Gly Leu Pro Lys Glu Ala Cys Met Glu Ile
   515 520 525
   Ser Ser Pro Gly Cys Gln Pro Pro Ala Gln Arg Leu Cys Gln Pro Val
   530 535 540
   Leu Pro Ser Pro Ala Cys Gln Leu Val Leu His Gln Ile Leu Lys Gly
   545 550 555 560
   Gly Ser Gly Thr Tyr Cys Leu Asn Val Ser Leu Ala Asp Thr Asn Ser
   565 570 575
   Leu Ala Val Val Ser Thr Gln Leu Ile Met Pro Gly Gln Glu Ala Gly
   580 585 590
   Leu Gly Gln Val Pro Leu Ile Val Gly Ile Leu Leu Val Leu Met Ala
   595 600 605
   Val Val Leu Ala Ser Leu Ile Tyr Arg Arg Arg Leu Met Lys Gln Asp
   610 615 620
   Phe Ser Val Pro Gln Leu Pro His Ser Ser Ser His Trp Leu Arg Leu
   625 630 635 640
   Pro Arg Ile Phe Cys Ser Cys Pro Ile Gly Glu Asn Ser Pro Leu Leu
   645 650 655
   Ser Gly Gln Gln Val
   660
<210> 3
   <211> 15
   <212> PRT
   <213> Artificial
<220>
   <223> gp 100 peptide
   <400> 3
   His Leu Ala Val Ile Gly Ala Leu Leu Ala Val Gly Ala Thr Lys
   1 5 10 15
<210> 4
   <211> 15
   <212> PRT
   <213> Artificial
<220>
   <223> gp 100 peptide
   <400> 4
   Gly Ala Leu Leu Ala Val Gly Ala Thr Lys Val Pro Arg Asn Gln
   1 5 10 15
<210> 5
   <211> 15
   <212> PRT
   <213> Artificial
<220>
   <223> gp 100 peptide
   <400> 5
   Val Gly Ala Thr Lys Val Pro Arg Asn Gln Asp Trp Leu Gly Val
   1 5 10 15
<210> 6
   <211> 15
   <212> PRT
   <213> Artificial
<220>
   <223> gp 100 peptide
   <400> 6
   Val Pro Arg Asn Gln Asp Trp Leu Gly Val Ser Arg Gln Leu Arg
   1 5 10 15
<210> 7
   <211> 15
   <212> PRT
   <213> Artificial
<220>
   <223> gp 100 peptide
   <400> 7
   Asp Trp Leu Gly Val Ser Arg Gln Leu Arg Thr Lys Ala Trp Asn
   1 5 10 15
<210> 8
   <211> 15
   <212> PRT
   <213> Artificial
<220>
   <223> gp 100 peptide
   <400> 8
   Ser Arg Gln Leu Arg Thr Lys Ala Trp Asn Arg Gln Leu Tyr Pro
   1 5 10 15
<210> 9
   <211> 15
   <212> PRT
   <213> Artificial
<220>
   <223> gp 100 peptide
   <400> 9
   Thr Lys Ala Trp Asn Arg Gln Leu Tyr Pro Glu Trp Thr Glu Ala
   1 5 10 15
<210> 10
   <211> 15
   <212> PRT
   <213> Artificial
<220>
   <223> gp 100 peptide
   <400> 10
   Arg Gln Leu Tyr Pro Glu Trp Thr Glu Ala Gln Arg Leu Asp Cys
   1 5 10 15
<210> 11
   <211> 15
   <212> PRT
   <213> Artificial
<220>
   <223> gp 100 peptide
   <400> 11
   Glu Trp Thr Glu Ala Gln Arg Leu Asp Cys Trp Arg Gly Gly Gln
   1 5 10 15
<210> 12
   <211> 15
   <212> PRT
   <213> Artificial
<220>
   <223> gp 100 peptide
   <400> 12
   Gln Arg Leu Asp Cys Trp Arg Gly Gly Gln Val Ser Leu Lys Val
   1 5 10 15
<210> 13
   <211> 15
   <212> PRT
   <213> Artificial
<220>
   <223> gp 100 peptide
   <400> 13
   Trp Arg Gly Gly Gln Val Ser Leu Lys Val Ser Asn Asp Gly Pro
   1 5 10 15
<210> 14
   <211> 15
   <212> PRT
   <213> Artificial
<220>
   <223> gp 100 peptide
   <400> 14
   Val Ser Leu Lys Val Ser Asn Asp Gly Pro Thr Leu Ile Gly Ala
   1 5 10 15
<210> 15
   <211> 15
   <212> PRT
   <213> Artificial
<220>
   <223> gp 100 peptide
   <400> 15
   Ile Ala Leu Asn Phe Pro Gly Ser Gln Lys Val Leu Pro Asp Gly
   1 5 10 15
<210> 16
   <211> 15
   <212> PRT
   <213> Artificial
<220>
   <223> gp 100 peptide
   <400> 16
   Pro Gly Ser Gln Lys Val Leu Pro Asp Gly Gln Val Ile Trp Val
   1 5 10 15
<210> 17
   <211> 15
   <212> PRT
   <213> Artificial
<220>
   <223> gp 100 peptide
   <400> 17
   Val Leu Pro Asp Gly Gln Val Ile Trp Val Asn Asn Thr Ile Ile
   1 5 10 15
<210> 18
   <211> 15
   <212> PRT
   <213> Artificial
<220>
   <223> gp 100 peptide
   <400> 18
   Gln Val Ile Trp Val Asn Asn Thr Ile Ile Asn Gly Ser Gln Val
   1 5 10 15
<210> 19
   <211> 15
   <212> PRT
   <213> Artificial
<220>
   <223> gp 100 peptide
   <400> 19
   Asn Asn Thr Ile Ile Asn Gly Ser Gln Val Trp Gly Gly Gln Pro
   1 5 10 15
<210> 20
   <211> 15
   <212> PRT
   <213> Artificial
<220>
   <223> gp 100 peptide
   <400> 20
   Asn Gly Ser Gln Val Trp Gly Gly Gln Pro Val Tyr Pro Gln Glu
   1 5 10 15
<210> 21
   <211> 15
   <212> PRT
   <213> Artificial
<220>
   <223> gp 100 peptide
   <400> 21
   Trp Gly Gly Gln Pro Val Tyr Pro Gln Glu Thr Asp Asp Ala Cys
   1 5 10 15
<210> 22
   <211> 15
   <212> PRT
   <213> Artificial
<220>
   <223> gp 100 peptide
   <400> 22
   Val Tyr Pro Gln Glu Thr Asp Asp Ala Cys Ile Phe Pro Asp Gly
   1 5 10 15
<210> 23
   <211> 15
   <212> PRT
   <213> Artificial
<220>
   <223> gp 100 peptide
   <400> 23
   Thr Asp Asp Ala Cys Ile Phe Pro Asp Gly Gly Pro Cys Pro Ser
   1 5 10 15
<210> 24
   <211> 15
   <212> PRT
   <213> Artificial
<220>
   <223> gp 100 peptide
   <400> 24
   Ile Phe Pro Asp Gly Gly Pro Cys Pro Ser Gly Ser Trp Ser Gln
   1 5 10 15
<210> 25
   <211> 15
   <212> PRT
   <213> Artificial
<220>
   <223> gp 100 peptide
   <400> 25
   Gly Ser Trp Ser Gln Lys Arg Ser Phe Val Tyr Val Trp Lys Thr
   1 5 10 15
<210> 26
   <211> 15
   <212> PRT
   <213> Artificial
<220>
   <223> gp 100 peptide
   <400> 26
   Lys Arg Ser Phe Val Tyr Val Trp Lys Thr Trp Gly Gln Tyr Trp
   1 5 10 15
<210> 27
   <211> 15
   <212> PRT
   <213> Artificial
<220>
   <223> gp 100 peptide
   <400> 27
   Tyr Val Trp Lys Thr Trp Gly Gln Tyr Trp Gln Val Leu Gly Gly
   1 5 10 15
<210> 28
   <211> 15
   <212> PRT
   <213> Artificial
<220>
   <223> gp 100 peptide
   <400> 28
   Trp Gly Gln Tyr Trp Gln Val Leu Gly Gly Pro Val Ser Gly Leu
   1 5 10 15
<210> 29
   <211> 15
   <212> PRT
   <213> Artificial
<220>
   <223> gp 100 peptide
   <400> 29
   Gln Val Leu Gly Gly Pro Val Ser Gly Leu Ser Ile Gly Thr Gly
   1 5 10 15
<210> 30
   <211> 15
   <212> PRT
   <213> Artificial
<220>
   <223> gp 100 peptide
   <400> 30
   Pro Val Ser Gly Leu Ser Ile Gly Thr Gly Arg Ala Met Leu Gly
   1 5 10 15
<210> 31
   <211> 15
   <212> PRT
   <213> Artificial
<220>
   <223> gp 100 peptide
   <400> 31
   Ser Ile Gly Thr Gly Arg Ala Met Leu Gly Thr His Thr Met Glu
   1 5 10 15
<210> 32
   <211> 15
   <212> PRT
   <213> Artificial
<220>
   <223> gp 100 peptide
   <400> 32
   Arg Ala Met Leu Gly Thr His Thr Met Glu Val Thr Val Tyr His
   1 5 10 15
<210> 33
   <211> 15
   <212> PRT
   <213> Artificial
<220>
   <223> gp 100 peptide
   <400> 33
   Thr His Thr Met Glu Val Thr Val Tyr His Arg Arg Gly Ser Arg
   1 5 10 15
<210> 34
   <211> 15
   <212> PRT
   <213> Artificial
<220>
   <223> gp 100 peptide
   <400> 34
   Val Thr Val Tyr His Arg Arg Gly Ser Arg Ser Tyr Val Pro Leu
   1 5 10 15
<210> 35
   <211> 15
   <212> PRT
   <213> Artificial
<220>
   <223> gp 100 peptide
   <400> 35
   Arg Arg Gly Ser Arg Ser Tyr Val Pro Leu Ala His Ser Ser Ser
   1 5 10 15
<210> 36
   <211> 15
   <212> PRT
   <213> Artificial
<220>
   <223> gp 100 peptide
   <400> 36
   Ser Tyr Val Pro Leu Ala His Ser Ser Ser Ala Phe Thr Ile Thr
   1 5 10 15
<210> 37
   <211> 15
   <212> PRT
   <213> Artificial
<220>
   <223> gp 100 peptide
   <400> 37
   Ala Phe Thr Ile Thr Asp Gln Val Pro Phe Ser Val Ser Val Ser
   1 5 10 15
<210> 38
   <211> 15
   <212> PRT
   <213> Artificial
<220>
   <223> gp 100 peptide
   <400> 38
   Asp Gln Val Pro Phe Ser Val Ser Val Ser Gln Leu Arg Ala Leu
   1 5 10 15
<210> 39
   <211> 15
   <212> PRT
   <213> Artificial
<220>
   <223> gp 100 peptide
   <400> 39
   Ser Val Ser Val Ser Gln Leu Arg Ala Leu Asp Gly Gly Asn Lys
   1 5 10 15
<210> 40
   <211> 15
   <212> PRT
   <213> Artificial
<220>
   <223> gp 100 peptide
   <400> 40
   Asp Gly Gly Asn Lys His Phe Leu Arg Asn Gln Pro Leu Thr Phe
   1 5 10 15
<210> 41
   <211> 15
   <212> PRT
   <213> Artificial
<220>
   <223> gp 100 peptide
   <400> 41
   His Phe Leu Arg Asn Gln Pro Leu Thr Phe Ala Leu Gln Leu His
   1 5 10 15
<210> 42
   <211> 15
   <212> PRT
   <213> Artificial
<220>
   <223> gp 100 peptide
   <400> 42
   Gln Pro Leu Thr Phe Ala Leu Gln Leu His Asp Pro Ser Gly Tyr
   1 5 10 15
<210> 43
   <211> 15
   <212> PRT
   <213> Artificial
<220>
   <223> gp 100 peptide
   <400> 43
   Ala Leu Gln Leu His Asp Pro Ser Gly Tyr Leu Ala Glu Ala Asp
   1 5 10 15
<210> 44
   <211> 15
   <212> PRT
   <213> Artificial
<220>
   <223> gp 100 peptide
   <400> 44
   Asp Phe Gly Asp Ser Ser Gly Thr Leu Ile Ser Arg Ala Leu Val
   1 5 10 15
<210> 45
   <211> 15
   <212> PRT
   <213> Artificial
<220>
   <223> gp 100 peptide
   <400> 45
   Ser Thr Gly Leu Ile Ser Arg Ala Leu Val Val Thr His Thr Tyr
   1 5 10 15
<210> 46
   <211> 15
   <212> PRT
   <213> Artificial
<220>
   <223> gp 100 peptide
   <400> 46
   Ser Arg Ala Leu Val Val Thr His Thr Tyr Leu Glu Pro Gly Pro
   1 5 10 15
<210> 47
   <211> 15
   <212> PRT
   <213> Artificial
<220>
   <223> gp 100 peptide
   <400> 47
   Val Thr His Thr Tyr Leu Glu Pro Gly Pro Val Thr Ala Gln Val
   1 5 10 15
<210> 48
   <211> 15
   <212> PRT
   <213> Artificial
<220>
   <223> gp 100 peptide
   <400> 48
   Leu Glu Pro Gly Pro Val Thr Ala Gln Val Val Leu Gln Ala Ala
   1 5 10 15
<210> 49
   <211> 15
   <212> PRT
   <213> Artificial
<220>
   <223> gp 100 peptide
   <400> 49
   Val Thr Ala Gln Val Val Leu Gln Ala Ala Ile Pro Leu Thr Ser
   1 5 10 15
<210> 50
   <211> 15
   <212> PRT
   <213> Artificial
<220>
   <223> gp 100 peptide
   <400> 50
   Val Leu Gln Ala Ala Ile Pro Leu Thr Ser Cys Gly Ser Ser Pro
   1 5 10 15
<210> 51
   <211> 15
   <212> PRT
   <213> Artificial
<220>
   <223> gp 100 peptide
   <400> 51
   Ile Pro Leu Thr Ser Cys Gly Ser Ser Pro Val Pro Gly Thr Thr
   1 5 10 15
<210> 52
   <211> 15
   <212> PRT
   <213> Artificial
<220>
   <223> gp 100 peptide
   <400> 52
   Val Pro Gly Thr Thr Asp Gly His Arg Pro Thr Ala Glu Ala Pro
   1 5 10 15
<210> 53
   <211> 15
   <212> PRT
   <213> Artificial
<220>
   <223> gp 100 peptide
   <400> 53
   Asp Gly His Arg Pro Thr Ala Glu Ala Pro Asn Thr Thr Ala Gly
   1 5 10 15
<210> 54
   <211> 15
   <212> PRT
   <213> Artificial
<220>
   <223> gp 100 peptide
   <400> 54
   Thr Ala Glu Ala Pro Asn Thr Thr Ala Gly Gln Val Pro Thr Thr
   1 5 10 15
<210> 55
   <211> 15
   <212> PRT
   <213> Artificial
<220>
   <223> gp 100 peptide
   <400> 55
   Gln Val Pro Thr Thr Glu Val Val Gly Thr Thr Pro Gly Gln Ala
   1 5 10 15
<210> 56
   <211> 15
   <212> PRT
   <213> Artificial
<220>
   <223> gp 100 peptide
   <400> 56
   Glu Val Val Gly Thr Thr Pro Gly Gln Ala Pro Thr Ala Glu Pro
   1 5 10 15
<210> 57
   <211> 15
   <212> PRT
   <213> Artificial
<220>
   <223> gp 100 peptide
   <400> 57
   Thr Pro Gly Gln Ala Pro Thr Ala Glu Pro Ser Gly Thr Thr Ser
   1 5 10 15
<210> 58
   <211> 15
   <212> PRT
   <213> Artificial
<220>
   <223> gp 100 peptide
   <400> 58
   Pro Thr Ala Glu Pro Ser Gly Thr Thr Ser Val Gln Val Pro Thr
   1 5 10 15
<210> 59
   <211> 15
   <212> PRT
   <213> Artificial
<220>
   <223> gp 100 peptide
   <400> 59
   Ser Gly Thr Thr Ser Val Gln Val Pro Thr Thr Glu Val Ile Ser
   1 5 10 15
<210> 60
   <211> 15
   <212> PRT
   <213> Artificial
<220>
   <223> gp 100 peptide
   <400> 60
   Val Gln Val Pro Thr Thr Glu Val Ile Ser Thr Ala Pro Val Gln
   1 5 10 15
<210> 61
   <211> 15
   <212> PRT
   <213> Artificial
<220>
   <223> gp 100 peptide
   <400> 61
   Thr Glu Val Ile Ser Thr Ala Pro Val Gln Met Pro Thr Ala Glu
   1 5 10 15
<210> 62
   <211> 15
   <212> PRT
   <213> Artificial
<220>
   <223> gp 100 peptide
   <400> 62
   Thr Ala Pro Val Gln Met Pro Thr Ala Glu Ser Thr Gly Met Thr
   1 5 10 15
<210> 63
   <211> 15
   <212> PRT
   <213> Artificial
<220>
   <223> gp 100 peptide
   <400> 63
   Met Pro Thr Ala Glu Ser Thr Gly Met Thr Pro Glu Lys Val Pro
   1 5 10 15
<210> 64
   <211> 15
   <212> PRT
   <213> Artificial
<220>
   <223> gp 100 peptide
   <400> 64
   Ser Thr Gly Met Thr Pro Glu Lys Val Pro Val Ser Glu Val Met
   1 5 10 15
<210> 65
   <211> 15
   <212> PRT
   <213> Artificial
<220>
   <223> gp 100 peptide
   <400> 65
   Pro Glu Lys Val Pro Val Ser Glu Val Met Gly Thr Thr Leu Ala
   1 5 10 15
<210> 66
   <211> 15
   <212> PRT
   <213> Artificial
<220>
   <223> gp 100 peptide
   <400> 66
   Val Ser Glu Val Met Gly Thr Thr Leu Ala Glu Met Ser Thr Pro
   1 5 10 15
<210> 67
   <211> 15
   <212> PRT
   <213> Artificial
<220>
   <223> gp 100 peptide
   <400> 67
   Gly Thr Thr Leu Ala Glu Met Ser Thr Pro Glu Ala Thr Gly Met
   1 5 10 15
<210> 68
   <211> 15
   <212> PRT
   <213> Artificial
<220>
   <223> gp 100 peptide
   <400> 68
   Glu Met Ser Thr Pro Glu Ala Thr Gly Met Thr Pro Ala Glu Val
   1 5 10 15
<210> 69
   <211> 15
   <212> PRT
   <213> Artificial
<220>
   <223> gp 100 peptide
   <400> 69
   Ser Ile Val Val Leu Ser Gly Thr Thr Ala Ala Gln Val Thr Thr
   1 5 10 15
<210> 70
   <211> 15
   <212> PRT
   <213> Artificial
<220>
   <223> gp 100 peptide
   <400> 70
   Ser Gly Thr Thr Ala Ala Gln Val Thr Thr Thr Glu Trp Val Glu
   1 5 10 15
<210> 71
   <211> 15
   <212> PRT
   <213> Artificial
<220>
   <223> gp 100 peptide
   <400> 71
   Ala Gln Val Thr Thr Thr Glu Trp Val Glu Thr Thr Ala Arg Glu
   1 5 10 15
<210> 72
   <211> 15
   <212> PRT
   <213> Artificial
<220>
   <223> gp 100 peptide
   <400> 72
   Thr Glu Trp Val Glu Thr Thr Ala Arg Glu Leu Pro Ile Pro Glu
   1 5 10 15
<210> 73
   <211> 15
   <212> PRT
   <213> Artificial
<220>
   <223> gp 100 peptide
   <400> 73
   Thr Thr Ala Arg Glu Leu Pro Ile Pro Glu Pro Glu Gly Pro Asp
   1 5 10 15
<210> 74
   <211> 15
   <212> PRT
   <213> Artificial
<220>
   <223> gp 100 peptide
   <400> 74
   Leu Pro Ile Pro Glu Pro Glu Gly Pro Asp Ala Ser Ser Ile Met
   1 5 10 15
<210> 75
   <211> 15
   <212> PRT
   <213> Artificial
<220>
   <223> gp 100 peptide
   <400> 75
   Pro Glu Gly Pro Asp Ala Ser Ser Ile Met Ser Thr Glu Ser Ile
   1 5 10 15
<210> 76
   <211> 15
   <212> PRT
   <213> Artificial
<220>
   <223> gp 100 peptide
   <400> 76
   Ala Ser Ser Ile Met Ser Thr Glu Ser Ile Thr Gly Ser Leu Gly
   1 5 10 15
<210> 77
   <211> 15
   <212> PRT
   <213> Artificial
<220>
   <223> gp 100 peptide
   <400> 77
   Ser Thr Glu Ser Ile Thr Gly Ser Leu Gly Pro Leu Leu Asp Gly
   1 5 10 15
<210> 78
   <211> 15
   <212> PRT
   <213> Artificial
<220>
   <223> gp 100 peptide
   <400> 78
   Thr Gly Ser Leu Gly Pro Leu Leu Asp Gly Thr Ala Thr Leu Arg
   1 5 10 15
<210> 79
   <211> 15
   <212> PRT
   <213> Artificial
<220>
   <223> gp 100 peptide
   <400> 79
   Pro Leu Leu Asp Gly Thr Ala Thr Leu Arg Leu Val Lys Arg Gln
   1 5 10 15
<210> 80
   <211> 15
   <212> PRT
   <213> Artificial
<220>
   <223> gp 100 peptide
   <400> 80
   Thr Ala Thr Leu Arg Leu Val Lys Arg Gln Val Pro Leu Asp Cys
   1 5 10 15
<210> 81
   <211> 15
   <212> PRT
   <213> Artificial
<220>
   <223> gp 100 peptide
   <400> 81
   Leu Val Lys Arg Gln Val Pro Leu Asp Cys Val Leu Tyr Arg Tyr
   1 5 10 15
<210> 82
   <211> 15
   <212> PRT
   <213> Artificial
<220>
   <223> gp 100 peptide
   <400> 82
   Val Pro Leu Asp Cys Val Leu Tyr Arg Tyr Gly Ser Phe Ser Val
   1 5 10 15
<210> 83
   <211> 15
   <212> PRT
   <213> Artificial
<220>
   <223> gp 100 peptide
   <400> 83
   Val Leu Tyr Arg Tyr Gly Ser Phe Ser Val Thr Leu Asp Ile Val
   1 5 10 15
<210> 84
   <211> 15
   <212> PRT
   <213> Artificial
<220>
   <223> gp 100 peptide
   <400> 84
   Thr Leu Asp Ile Val Gln Gly Ile Glu Ser Ala Glu Ile Leu Gln
   1 5 10 15
<210> 85
   <211> 15
   <212> PRT
   <213> Artificial
<220>
   <223> gp 100 peptide
   <400> 85
   Gln Gly Ile Glu Ser Ala Glu Ile Leu Gln Ala Val Pro Ser Gly
   1 5 10 15
<210> 86
   <211> 15
   <212> PRT
   <213> Artificial
<220>
   <223> gp 100 peptide
   <400> 86
   Ala Glu Ile Leu Gln Ala Val Pro Ser Gly Glu Gly Asp Ala Phe
   1 5 10 15
<210> 87
   <211> 15
   <212> PRT
   <213> Artificial
<220>
   <223> gp 100 peptide
   <400> 87
   Ala Val Pro Ser Gly Glu Gly Asp Ala Phe Glu Leu Thr Val Ser
   1 5 10 15
<210> 88
   <211> 15
   <212> PRT
   <213> Artificial
<220>
   <223> gp 100 peptide
   <400> 88
   Glu Gly Asp Ala Phe Glu Leu Thr Val Ser Cys Gln Gly Gly Leu
   1 5 10 15
<210> 89
   <211> 15
   <212> PRT
   <213> Artificial
<220>
   <223> gp 100 peptide
   <400> 89
   Glu Leu Thr Val Ser Cys Gln Gly Gly Leu Pro Lys Glu Ala Cys
   1 5 10 15
<210> 90
   <211> 15
   <212> PRT
   <213> Artificial
<220>
   <223> gp 100 peptide
   <400> 90
   Cys Gln Gly Gly Leu Pro Lys Glu Ala Cys Met Glu Ile Ser Ser
   1 5 10 15
<210> 91
   <211> 15
   <212> PRT
   <213> Artificial
<220>
   <223> gp 100 peptide
   <400> 91
   Pro Lys Glu Ala Cys Met Glu Ile Ser Ser Pro Gly Cys Gln Pro
   1 5 10 15
<210> 92
   <211> 15
   <212> PRT
   <213> Artificial
<220>
   <223> gp 100 peptide
   <400> 92
   Met Glu Ile Ser Ser Pro Gly Cys Gln Pro Pro Ala Gln Arg Leu
   1 5 10 15
<210> 93
   <211> 15
   <212> PRT
   <213> Artificial
<220>
   <223> gp 100 peptide
   <400> 93
   Pro Ala Gln Arg Leu Cys Gln Pro Val Leu Pro Ser Pro Ala Cys
   1 5 10 15
<210> 94
   <211> 15
   <212> PRT
   <213> Artificial
<220>
   <223> gp 100 peptide
   <400> 94
   Cys Gln Pro Val Leu Pro Ser Pro Ala Cys Gln Leu Val Leu His
   1 5 10 15
<210> 95
   <211> 15
   <212> PRT
   <213> Artificial
<220>
   <223> gp 100 peptide
   <400> 95
   Pro Ser Pro Ala Cys Gln Leu Val Leu His Gln Ile Leu Lys Gly
   1 5 10 15
<210> 96
   <211> 15
   <212> PRT
   <213> Artificial
<220>
   <223> gp 100 peptide
   <400> 96
   Gln Leu Val Leu His Gln Ile Leu Lys Gly Gly Ser Gly Thr Tyr
   1 5 10 15
<210> 97
   <211> 15
   <212> PRT
   <213> Artificial
<220>
   <223> gp 100 peptide
   <400> 97
   Leu Ala Asp Thr Asn Ser Leu Ala Val Val Ser Thr Gln Leu Ile
   1 5 10 15
<210> 98
   <211> 15
   <212> PRT
   <213> Artificial
<220>
   <223> gp 100 peptide
   <400> 98
   Ser Leu Ala Val Val Ser Thr Gln Leu Ile Met Pro Gly Gln Glu
   1 5 10 15
<210> 99
   <211> 15
   <212> PRT
   <213> Artificial
<220>
   <223> gp 100 peptide
   <400> 99
   Ser Thr Gln Leu Ile Met Pro Gly Gln Glu Ala Gly Leu Gly Gln
   1 5 10 15
<210> 100
   <211> 15
   <212> PRT
   <213> Artificial
<220>
   <223> gp 100 peptide
   <400> 100
   Met Pro Gly Gln Glu Ala Gly Leu Gly Gln Val Pro Leu Ile Val
   1 5 10 15
<210> 101
   <211> 15
   <212> PRT
   <213> Artificial
<220>
   <223> gp 100 peptide
   <400> 101
   Ala Gly Leu Gly Gln Val Pro Leu Ile Val Gly Ile Leu Leu Val
   1 5 10 15
<210> 102
   <211> 15
   <212> PRT
   <213> Artificial
<220>
   <223> gp 100 peptide
   <400> 102
   Leu Met Ala Val Val Leu Ala Ser Leu Ile Tyr Arg Arg Arg Leu
   1 5 10 15
<210> 103
   <211> 15
   <212> PRT
   <213> Artificial
<220>
   <223> gp 100 peptide
   <400> 103
   Leu Met Ala Val Val Leu Ala Ser Leu Ile Tyr Arg Arg Arg Leu
   1 5 10 15
<210> 104
   <211> 15
   <212> PRT
   <213> Artificial
<220>
   <223> gp 100 peptide
   <400> 104
   Met Lys Gln Asp Phe Ser Val Pro Gln Leu Pro His Ser Ser Ser
   1 5 10 15
<210> 105
   <211> 15
   <212> PRT
   <213> Artificial
<220>
   <223> gp 100 peptide
   <400> 105
   Ser Val Pro Gln Leu Pro His Ser Ser Ser His Trp Leu Arg Leu
   1 5 10 15
<210> 106
   <211> 15
   <212> PRT
   <213> Artificial
<220>
   <223> gp 100 peptide
   <400> 106
   Pro His Ser Ser Ser His Trp Leu Arg Leu Pro Arg Ile Phe Cys
   1 5 10 15
<210> 107
   <211> 15
   <212> PRT
   <213> Artificial
<220>
   <223> gp 100 peptide
   <400> 107
   His Trp Leu Arg Leu Pro Arg Ile Phe Cys Ser Cys Pro Ile Gly
   1 5 10 15
<210> 108
   <211> 15
   <212> PRT
   <213> Artificial
<220>
   <223> gp 100 peptide
   <400> 108
   Pro Arg Ile Phe Cys Ser Cys Pro Ile Gly Glu Asn Ser Pro Leu
   1 5 10 15
<210> 109
   <211> 59
   <212> DNA
   <213> Artificial
<220>
   <223> oligo MPSYN 763
   <400> 109
   ccctctagat cgcgatatcc gttaagtttg tatcgtaatg cttgcatttt gttattcgt 59
<210> 110
   <211> 31
   <212> DNA
   <213> Artificial
<220>
   <223> oligo MPSYN 764
   <400> 110
   cccgaattca taaaaattat tgatgtctac a 31
<210> 111
   <211> 23
   <212> DNA
   <213> Artificial
<220>
   <223> oligo SPCSPL1
   <400> 111
   gatcgtcgac gagctcgaat tcg 23
<210> 112
   <211> 23
   <212> DNA
   <213> Artificial
<220>
   <223> oligo SPC5PL2
   <400> 112
   gatccgaatt cgagctcgtc gac 23
<210> 113
   <211> 51
   <212> DNA
   <213> Artificial
<220>
   <223> oligo MELgp01
   <400> 113
   ccctcgcgat atccgttaag tttgtatcgt aatggatctg gtgctaaaaag 51
<210> 114
   <211> 36
   <212> DNA
   <213> Artificial
<220>
   <223> oligo MELgp02
   <400> 114
   cccctcgaga taaaaatcag acctgctgcc cactga 36
<210> 115
   <211> 18
   <212> DNA
   <213> Artificial
<220>
   <223> oligo MELgp05
   <400> 115
   cccatctggc tcttggtc 18
<210> 116
   <211> 21
   <212> DNA
   <213> Artificial
<220>
   <223> oligo MELgp13
   <400> 116
   tgacatctct gccagtgtgg t 21
<210> 117
   <211> 38
   <212> DNA
   <213> Artificial
<220>
   <223> Primer 209-A
   <400> 117
   gctcagcctt caccattatg gaccaggtgc ctttctcc 38
<210> 118
   <211> 38
   <212> DNA
   <213> Artificial
<220>
   <223> Primer 209-B
   <400> 118
   ggagaaaggc acctggtcca taatggtgaa ggctgacg 38
<210> 119
   <211> 38
   <212> DNA
   <213> Artificial
<220>
   <223> Primer 280-A
   <400> 119
   gagcctggcc cagtcactgt tcaggtggtc ctgcaggc 38
<210> 120
   <211> 38
   <212> DNA
   <213> Artificial
<220>
   <223> Primer 280-B
   <400> 120
   gcctgcagga ccacctgaac agtgactggg ccaggctc 38
<210> 121
   <211> 59
   <212> DNA
   <213> Artificial
<220>
   <223> Primer MEL25
   <400> 121
   gctccgggat ccccggcgat ggtagacagt cacttccatc gtgtgtgtgc ccagcattg 59
<210> 122
   <211> 59
   <212> DNA
   <213> Artificial
<220>
   <223> Primer MEL27
   <400> 122
   atcgcgatat ccgttaagtt tgtatcgtaa tggatctggt gctaaaaaga tgccttctt 59
<210> 123
   <211> 2534
   <212> DNA
   <213> Artificial
<220>
   <223> modified gp 100
   <400> 123
   ggctactttt caacaaagga gcagatgtaa actacatctt tgaaagaaat ggaaaatcat     60
   atactgtttt ggaattgatt aaagaaagtt actctgagac acaaaagagg tagctgaagt    120
   ggtactctca aaggtacgtg actaattagc tataaaaagg atcgtcgacg agctcgaatt    180
   cggatccggg ttaattaatt agtcatcagg cagggcgaga acgagactat ctgctcgtta    240
   attaattaga gcttctttat tctatactta aaaagtgaaa ataaatacaa aggttcttga    300
   gggttgtgtt aaattgaaag cgagaaataa tcataaatta tttcattatc gcgatatccg    360
   ttaagtttgt atcgtaatgg atctggtgct aaaaagatgc cttcttcatt tggctgtgat    420
   aggtgctttg ctggctgtgg gggctacaaa agtacccaga aaccaggact ggcttggtgt    480
   ctcaaggcaa ctcagaacca aagcctggaa caggcagctg tatccagagt ggacagaagc    540
   ccagagactt gactgctgga gaggtggtca agtgtccctc aaggtcagta atgatgggcc 600
   tacactgatt ggtgcaaatg cctccttctc tattgccttg aacttccctg gaagccaaaa 660
   ggtattgcca gatgggcagg ttatctgggt caacaatacc atcatcaatg ggagccaggt 720
   gtggggagga cagccagtgt atccccagga aactgacgat gcctgcatct tccctgatgg 780
   tggaccttgc ccatctggct cttggtctca gaagagaagc tttgtttatg tctggaagac 840
   ctggggccaa tactggcaag ttctaggggg cccagtgtct gggctgagca ttgggacagg 900
   cagggcaatg ctgggcacac acacgatgga agtgactgtc taccatcgcc ggggatcccg 960
   gagctatgtg cctcttgctc attccagctc agccttcacc attatggacc aggtgccttt 1020
   ctccgtgagc gtgtcccagt tgcgggcctt ggatggaggg aacaagcact tcctgagaaa 1080
   tcagcctctg acctttgccc tccagctcca tgaccccagt ggctatctgg ctgaagctga 1140
   cctctcctac acctgggact ttggagacag tagtggaacc ctgatctctc gggcacttgt 1200
   ggtcactcat acttacctgg agcctggccc agtcactgtt caggtggtcc tgcaggctgc 1260
   cattcctctc acctcctgtg gctcctcccc agttccaggc accacagatg ggcacaggcc 1320
   aactgcagag gcccctaaca ccacagctgg ccaagtgcct actacagaag ttgtgggtac 1380
   tacacctggt caggcgccaa ctgcagagcc ctctggaacc acatctgtgc aggtgccaac 1440
   cactgaagtc ataagcactg cacctgtgca gatgccaact gcagagagca caggtatgac 1500
   acctgagaag gtgccagttt cagaggtcat gggtaccaca ctggcagaga tgtcaactcc 1560
   agaggctaca ggtatgacac ctgcagaggt atcaattgtg gtgctttctg gaaccacagc 1620
   tgcacaggta acaactacag agtgggtgga gaccacagct agagagctac ctatccctga 1680
   gcctgaaggt ccagatgcca gctcaatcat gtctacggaa agtattacag gttccctggg 1740
   ccccctgctg gatggtacag ccaccttaag gctggtgaag agacaagtcc ccctggattg 1800
   tgttctgtat cgatatggtt ccttttccgt caccctggac attgtccagg gtattgaaag 1860
   tgccgagatc ctgcaggctg tgccgtccgg tgagggggat gcatttgagc tgactgtgtc 1920
   ctgccaaggc gggctgccca aggaagcctg catggagatc tcatcgccag ggtgccagcc 1980
   ccctgcccag cggctgtgcc agcctgtgct acccagccca gcctgccagc tggttctgca 2040
   ccagatactg aagggtggct cggggacata ctgcctcaat gtgtctctgg ctgataccaa 2100
   cagcctggca gtggtcagca cccagcttat catgcctggt caagaagcag gccttgggca 2160
   ggttccgctg atcgtgggca tcttgctggt gttgatggct gtggtccttg catctctgat 2220
   atataggcgc agacttatga agcaagactt ctccgtaccc cagttgccac atagcagcag 2280
   tcactggctg cgtctacccc gcatcttctg ctcttgtccc attggtgaga acagccccct 2340
   cctcagtggg cagcaggtct gatttttatc tcgagtctag aatcgatccc gggtttttat 2400
   gactagttaa tcacggccgc ttataaagat ctaaaatgca taatttctaa ataatgaaaa 2460
   aaaagtacat catgagcaac gcgttagtat attttacaat ggagattaac gctctatacc 2520
   gttctatgtt tatt 2534
<210> 124
   <211> 9
   <212> PRT
   <213> Artificial
<220>
   <223> gp 100 peptide
   <400> 124
   Ile Met Asp Gln Val Pro Phe Ser Tyr
   1 5
<210> 125
   <211> 9
   <212> PRT
   <213> Artificial
<220>
   <223> gp 100 peptide
   <400> 125
   Tyr Leu Glu Pro Gly Pro Val Thr Val
   1 5